(19)

Europäisches
Patentamt
European
Patent Office
Office européen
des brevets

(11) **EP 2 514 407 A1**

(12) **EUROPEAN PATENT APPLICATION**

(43) Date of publication:
24.10.2012 Bulletin 2012/43

(21) Application number: 12159419.6

(22) Date of filing: 30.05.2008

(51) Int Cl.:
*A61K 9/00* (2006.01)     *A61K 9/08* (2006.01)
*A61K 9/19* (2006.01)     *A61K 38/04* (2006.01)
*A61K 38/16* (2006.01)     *A61K 47/10* (2006.01)
*A61K 47/42* (2006.01)     *A61K 9/12* (2006.01)
*A61K 38/28* (2006.01)

(84) Designated Contracting States:
AT BE BG CH CY CZ DE DK EE ES FI FR GB GR
HR HU IE IS IT LI LT LU LV MC MT NL NO PL PT
RO SE SI SK TR

(30) Priority: 01.06.2007 EP 07109435
17.08.2007 EP 07114524

(62) Document number(s) of the earlier application(s) in
accordance with Art. 76 EPC:
08760278.5 / 2 164 458

(71) Applicant: **Novo Nordisk A/S**
**2880 Bagsværd (DK)**

(72) Inventors:
• **Bjerregaard Jensen, Simon**
**3880 Bagsvaerd (DK)**
• **Havelund, Svend**
**2880 Bagsvaerd (DK)**
• **Föger, Florian Anders**
**2880 Bagsvaerd (DK)**

Remarks:
•This application was filed on 14-03-2012 as a
divisional application to the application mentioned
under INID code 62.
•Claims filed after the date of receipt of the divisional
application (Rule 68(4) EPC).

(54) **Stable non-aqueous pharmaceutical compositions**

(57)     The present invention relates to shelf stable non-aqueous pharmaceutical compositions comprising a dehydrated therapeutically active polypeptide comprising 10-100 amino acids, at least one semi-polar protic organic solvent and which polypetide has been dehydrated at a target pH which is at least 1 pH unit distant from the pI. The composition can be used in methods of treating diabetes, hyperglycaemia and impaired glucose tolerance.

**Description**

**FIELD OF THE INVENTION**

The present invention relates to non-aqueous pharmaceutical compositions, and to the use thereof in methods of treating diabetes and hyperglycaemia.

**BACKGROUND OF INVENTION**

Polypeptides have typically low solubilities in most non-aqueous solvents except certain aprotic solvents like dimethyl sulfoxide (DMSO), dimethylacetamide and dimethylformamide. However, the toxicity of these aprotic solvents disqualifies them from being used in pharmaceutical formulations to any significant degree.

Klibanov et al (J. T. Chin, S. L. Wheeler, and A. M. Klibanov. Communication to the editor: On protein solubility in organic solvents. BIOTECHNOL.BIOENG. 44 (1):140-145, 1994) describesthat protic, very hydrophilic and polar solvents dissolve more than 10 mg/ml lysozyme(lyophilized fromaqueous solution of pH 6.0). The solubility in an individual solvent (1,5pentanediol) markedly increased when the lysozyme was freeze dried prior to dissolution in pentanediolfrom aqueous solutions where pH values were moved below the isoelectric point of lysozyme. No strong correlation between solvent characteristics and solubility of lysozyme could be established.

WO 00/42993 describes formulations for delivery of macromolecules, where the macromolecules are dissolved or dispersed in a low toxicity organic solvent which can be aerosolized for delivery to a patient's lung by inhalation.

Many organic polar protic solvents tend todestabilize polypeptides due to partial unfolding of the polypeptides, which often enhances aggregation and chemical degradation processes significantly due to higher conformational flexibility. Some organic polar protic solvents like ethanol might even act as a denaturant for polypeptides. Furthermore, the organic polar protic solvents often contain small amounts of highly reactive impurities such as aldehydes and ketones, which compromises the stability of the polypeptide. Hence, it is difficult to formulate a non-aqueous solution of a polypeptide with adequate shelf life stability.

Because non-aqueous polypeptide solutions can be further processed into solution pressurized metered dose inhalers (pMDI), where the polar non-aqueous act as a cosolvent in order for the polypeptide to be solubilized in ahydrofluoroalkane they can be advantageously used for pulmonaladministration.

The non-aqueous polypeptide solutions can also be processed into microemulsions for oral administration by adding detergents and non-polar hydrophobic solvents such as oils. The microemulsion might protect the polypeptide against proteolytic degradation and enhance the systemic absorption of the polypeptide from the gastrointestinal tract. Furthermore, it is anticipated that hydrolysis of the polypeptides is minimized in non-aqueous formulations due to lower water activity.

Various treatments and addition of excipients must often be applied to non-aqueous pharmaceutical compositions of therapeutic peptides in order to improve their solubility and their stability.

Shelf life of liquid parenteral formulations of peptides must be at least a year, preferably longer. The in-use period where the product may be transported and shaken daily at ambient temperature preferably should be several weeks.

Thus, there is a need for non-aqueous pharmaceutical compositions of therapeutic peptides which have improved stability.

**BRIEF DESCRIPTION OF THE DRAWINGS**

Figure 1. Relation between solubility of insulin aspart in propylene glycol at ambient temperature as a function of the pH of insulin aspart reconstituted in water (target pH).

Figure 2. Solubility of insulin aspart (target pH: 7.5) in various semi-polar protic solvents at ambient temperature.

Figure 3. Permeated Human Insulin in presence or absence of 10% (v/v) propylene glycol (PG) through freshly excised rat gut sacs (proximal jejunum) after 2 hours of incubation in physiological culture medium (pH 7.4) at 37°C.

Figure 4. The MALDI analyses shows permeated non-degraded insulin aspart (peak ~5828) and its degradation product (peak ~5218) in each of two buffer control samples (S3 and S6) and in two propylene glycol containing samples (SPG5 and SPG6). It can be seen that in presence of propylene glycol (PG)more non-degraded insulin permeates across intestinal mucosa.

Figure 5. Decrease of blood glucose (mmol/l) after per oral administration of insulin A14GluB25HisdesB30 human insulin(8 mM) with or without propylene glycol. The dosage volume of 1.2 ml/kg has been administrated to SPRD rats by using a gavage.

Figure 6. FUV CD of 0.2 mM InsulinAspart, pH 7.4 (full line), of 0.2 mM InsulinAspart in 100 % propylene glycol (PG) (dotted line), 0.2 mM InsulinAspart in 2 % PG after dilution from 100% propylene glycol (PG) (broken line).

Figure 7. NUV CD of 0.2 mM InsulinAspart, pH 7.4 (full line), of 0.2 mM InsulinAspart in 100 % propylene glycol (PG) (dotted line), 0.2 mM InsulinAspart in 2 % propylene glycol (PG) after dilution from 100% propylene glycol (PG) (broken line).

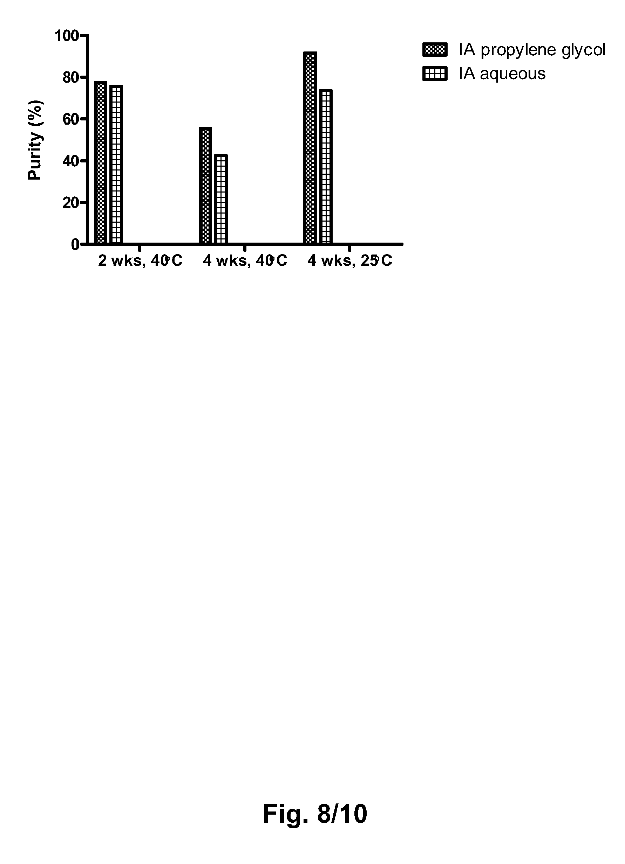

Figure 8. Purity of insulin aspart (IA) solubilized in either propylene glycol or 0.1 M TRIS buffer, pH 7.5, after incubation at 25 and 40°C for up to 4 weeks. Target pH of the insulin aspart powder was 7.5. Purity was determined with reverse phase chromatography.

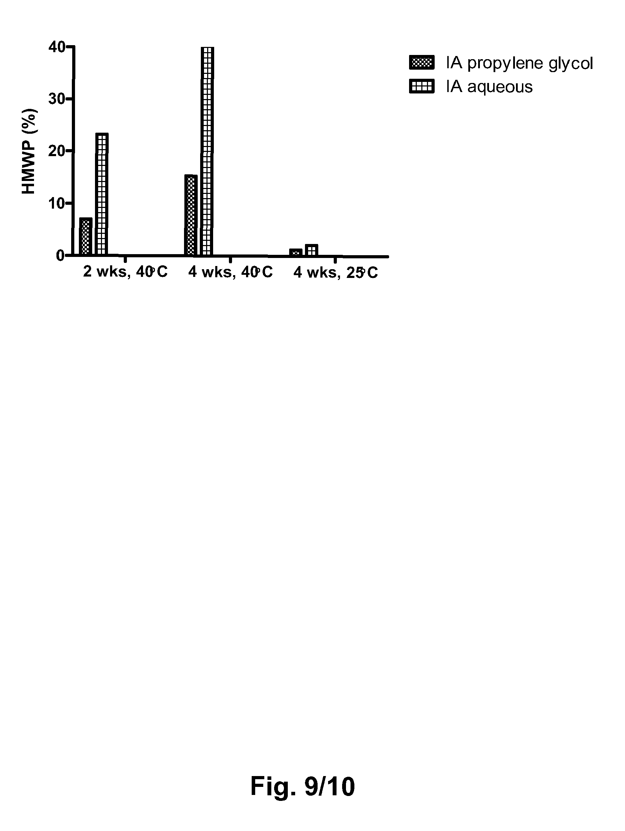

Figure 9. Formation of high molecular weight protein (HMWP) of insulin aspart (IA) solubilized in either propylene glycol or 0.1 M TRIS buffer, pH 7.5, after incubation at 25 and 40°C for up to 4 weeks. Target pH of the insulin aspart powder was 7.5. The amount of HMWP was determined with size exclusion chromatography.

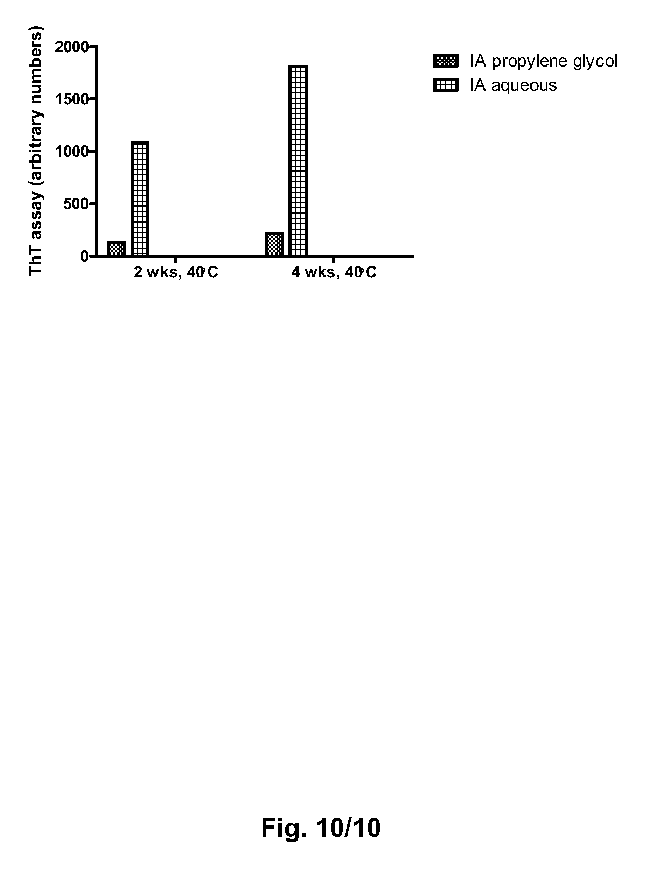

Figure 10. Formation of Thioflavin T positive fibrils of insulin aspart (IA) solubilized in either propylene glycol or 0.1 M TRIS buffer, pH 7.5, after incubation at 40°C for up to 4 weeks. Target pH of the insulin aspart powder was 7.5.

## SUMMARY OF THE INVENTION

In one aspect of the invention, a pharmaceutical non-aqueous composition comprising a dehydrated polypeptide, andat least onesemi-polar protic organic solventwhich polypeptide has been dehydratedat a target pH which is at least 1 pH unit from the pIof the polypeptide in aqueous solution, is provided.

In one aspect of the invention, a pharmaceutical non-aqueous composition comprising a dehydrated polypeptide, andat least onesemi-polar protic organic solventwhich polypeptide has been dehydratedat a target pH which is at least 1 pH unit from the pIof the polypeptide in aqueous solution, and where said target pH is in the range from about 6.0 to about 9.0, is provided.

## DETAILED DESCRIPTION OF THE INVENTION

We have discovered that polypeptides such as insulin peptides in solid state (dehydrated) can be solubilized to a very high degree in a non-aqueous semi-polar protic solvent by optimizing the pH of said polypeptides in an aqueous solutionbefore dehydration making the formulationof shelf-stable pharmaceutical formulations possible. Such pharmaceutical compositions for e.g. oral, pulmonal and nasal use show high chemical and/or physical stability. Compositions according to the invention such as oral pharmaceutical compositions using propylene glycol as a semi-polar protic organic solventmay also show significantly improved bioavailability.

In one aspect of the invention, a pharmaceutical non-aqueous composition comprising a dehydrated polypeptide, andat least onesemi-polar protic organic solventwhich polypeptide has been dehydratedat a target pH which is at least 1 pH unit from the pIof the polypeptide in aqueous solution, is provided.

In another aspect of the invention, a pharmaceutical non-aqueous composition comprising a dehydrated polypeptide, andat least onesemi-polar protic organic solventwhich polypeptide has been dehydratedat a target pH which is at least 1 pH unit from the pIof the polypeptide in aqueous solution, with the proviso that the polypeptide is not insulinotropic peptide, GLP-1(7-37) or an analog or derivative thereof, or exendin or an analog or derivative thereof, is provided.

In a further aspect of the invention, a pharmaceutical non-aqueous composition comprising

a) a dehydrated therapeutically active polypeptide, and
b) at least onesemi-polar protic organic solvent

which polypeptide has been dehydratedat a target pH which is at least 1 pH unit from the pIof the polypeptide in aqueous solution, and said target pH is in the range from about 6.0 to about 9.0, is provided.

In a yet further aspect of the invention, a pharmaceutical non-aqueous composition comprising

a) a dehydrated therapeutically active polypeptide, and

b) at least onesemi-polar protic organic solvent

which polypeptide has been dehydratedat a target pH which is at least 1 pH unit from the pIof the polypeptide in aqueous solution, and said target pH is in the range from about 6.0 to about 9.0, with the proviso that the polypeptide is not insulinotropic peptide, GLP-1(7-37) or an analog or derivative thereof, or exendin or an analog or derivative thereof, is provided.

In another aspect of the invention, a pharmaceutical non-aqueous composition comprising a mixture of

a) a dehydrated therapeutically active polypeptidecomprising 10-100 amino acids, and

b) at least onesemi-polar protic organic solvent

whichpolypeptide has been dehydratedat a target pH which is at least 1 pH unit from the pIof the polypeptide in aqueous solution. In a preferred embodiment, thetarget pH is in the range from about 6.0 to about 9.0.

In yet another aspect of the invention, a pharmaceutical non-aqueous composition comprising a mixture of

a) a dehydrated therapeutically active polypeptide comprising 10-100 amino acids, and

b) at least onesemi-polar protic organic solvent

whichpolypeptide has been dehydratedat a target pH which is at least 1 pH unit from the pIof the polypeptide in aqueous solution. In a preferred embodiment, thetarget pH is in the range from about 6.0 to about 9.0, with the proviso that the polypeptide is not insulinotropic peptide, GLP-1(7-37) or an analog or derivative thereof, or exendin or an analog or derivative thereof.

The term "non-aqueous" as used herein refers to a composition which comprises less than 10% w/w water. In a more preferred embodiment, the composition according to the invention comprises less than8% w/w water, in a more preferred embodiment less than 5% w/w water, in a more preferred embodiment less than 3% w/w water and in an even more preferred embodiment less than 2% w/w water.

The term "dehydrated" as used herein in connection with a polypeptide refers to a polypeptide which has been dried from an aqueous solution. The term "target pH" as used herein refers to the aqueous pH which will establish when dehydrated polypeptide is rehydrated in pure water to a concentration of approximately 40 mg/ml or more.The target pH will typically be identical to the pH of the aqueous polypeptide solution from which the polypeptide was recovered by drying. However, the pH of the polypeptide solution will not be identical to the target pH, if the polypeptide solution contains volatile acids or bases. It has been found that the pH history of the polypeptide will be determinant for the amount of the polypeptide, which can be solubilized in the semi-polar protic organic solvent.

According to the invention the polypeptide has been dehydratedat a target pH which is at least 1 pH unit from the pIof the polypeptide in aqueous solution. Thus, in one aspect of the invention, the target pH is more than 1 pH unit above the isoelectric point of the polypeptide. In another aspect of the invention, the target pH is more than 1 pH unit below the isoelectric point of the polypeptide. In a preferred aspect, the target pH is more than 1.5 pH units above or below the pI of the polypeptide. In an even more preferred aspect, the target pH is 2.0 pH units above or below the pI of the polypeptide. In a further aspect, the target pH is 2.5 pH units above or below the pI of the polypeptide. In yet a further aspect, the target pH is above the pI of the polypeptide.

By "volatile base" is meant a base, which to some extend will evaporate upon heating and/or at reduced pressure, e.g. bases which have a vapour pressure above 65 Pa at room temperature or an aqueous azeotropic mixture including a base having a vapour pressure above 65 Pa at room temperature. Examples of volatile bases are ammonium hydroxides, tetraalkylammonium hydroxides, secondary amines, tertiary amines, aryl amines, alphatic amines or ammonium bicarbonate or a combination. For example the volatile base can be bicarbonate, carbonate, ammonia, hydrazine or an organic base such as a lower aliphatic amines e.g. trimethyl amine, triethylamine, diethanolamines, triethanolamine and their salts. Further the volatile base can be ammonium hydroxide, ethyl amine or methyl amine or a combination hereof.

By "volatile acid" is meant an acid, which to some extend will evaporate upon heating and/or at reduced pressure, e.g. acids which have a vapour pressure above 65 Pa at room temperature or an aqueous azeotropic mixture including an acid having a vapour pressure above 65 Pa at room temperature. Examples of volatile acids are carbonic acid, formic acid, acetic acid, propionic acid and butyric acid.

A "non volatile base" as mentioned herein means a base, which do not evaporate or only partly evaporate upon heating, e.g. bases with a vapour pressure below 65 Pa at room temperature. The non volatile base can be selected from the group consisting of alkaline metal salts, alkaline metal hydroxides, alkaline earth metal salts, alkaline earth metal hydroxides and amino acids or a combination hereof. Examples of non-volatile bases are sodium hydroxide, potassium hydroxide, calcium hydroxide, and calcium oxide.

A "non volatile acid" as mentioned herein means an acid, which do not evaporate or only partly evaporate upon heating, e.g. bases with a vapour pressure below 65 Pa at room temperature. Examples of non-volatile acids are hydrochloric acid, phosphoric acid and sulfuric acid.

The term "the pIof the polypeptide" as used herein refers to the isoelectric point of a polypeptide.

The term "isoelectric point" as used herein means the pH value where the overall net charge of a macromolecule such as a peptide is zero. In peptides there may be several charged groups, and at the isoelectric point the sum of all these charges is zero. At a pH above the isoelectric point the overall net charge of the peptide will be negative, whereas at pH values below the isoelectric point the overall net charge of the peptide will be positive.

The pI of a protein can be determined experimentally by electrophoresis techniques such as electrofocusing:

A pH gradient is established in an anticonvective medium, such as a polyacrylamide gel. When a protein is introduced in to the system it will migrate under influence of an electric field applied across the gel. Positive charged proteins will migrate to the cathode. Eventually, the migrating protein reaches a point in the pH gradient where its net electrical charge is zero and is said to be focused. This is the isoelectric pH (pI) of the protein. The protein is then fixed on the gel and stained. The pI of the protein can then be determined by comparison of the position of the protein on the gel relative to marker molecules with known pI values.

The net charge of a protein at a given pH value can be estimated theoretically per a person skilled in the art by conventional methods. In essence, the net charge of protein is the equivalent to the sum of the fractional charges of the charged amino acids in the protein: aspartate ($\beta$-carboxyl group), glutamate ($\delta$-carboxyl group), cysteine (thiol group), tyrosine (phenol group), histidine (imidazole side chains), lysine ($\epsilon$-ammonium group) and arginine (guanidinium group). Additonally, one should also take into account charge of protein terminal groups ($\alpha$-NH$_2$ and $\alpha$-COOH). The fractional charge of the ionisable groups can be calculated from the intrinsic pKa values.

The drying i.e. dehydrationof the polypeptide can be performed by any conventional drying method such e.g. by spray-, freeze-, vacuum-, open - and contact drying. In one aspect of the invention, the polypeptide solution is spray dried to obtain a water content below about 10%. The water content may be below about 8%, below about 6%, below about 5%, below about 4%, below about 3%, below about 2% or below about 1% calculated on/measured by loss on drying test (gravimetric) as stated in the experimental part.

In one aspect of the invention the polypeptide is spray dried. In a further aspect of the invention, the polypeptide is freeze-dried.

In one aspect of the invention, the solubility obtained by the pre-treatment of the polypeptide by dehydration at the selected target pH in an organic solvent is at least 20 mg/ml. In a further aspect, the solubility of dehydrated polypeptide in the organic solvent is at least 30 mg/ml. In yet a further aspect,the solubility of dehydrated polypeptide in the organic solvent is at least 40 mg/ml. In yet a further aspect, the solubility of dehydrated polypeptide in the organic solvent is at least 50 mg/ml. In yet a further aspect,the solubility of dehydrated polypeptide in the organic solvent is at least 60 mg/ml. In yet a further aspect,the solubility of dehydrated polypeptide in the organic solvent is at least 70 mg/ml. In yet a further aspect,the solubility of dehydrated polypeptide in the organic solvent is at least 80 mg/ml. In yet a further aspect,the solubility of dehydrated polypeptide in the organic solvent is at least 100 mg/ml.

The term "semi-polar protic organic solvent" as used herein refers to a hydrophilic, water miscible carbon-containing solvent that contains an O-H or N-H bond, or mixtures thereof.The polarity is reflected in the dielectric constant or the dipole moment of a solvent. The polarity of a solvent determines what type of compounds it is able to dissolve and with what other solvents or liquid compounds it is miscible. Typically, polar solvents dissolve polar compounds best and non-polar solvents dissolve non-polar compounds best: "like dissolves like". Strongly polar compounds like inorganic salts (e.g. sodium chloride) dissolve only in very polar solvents.

Semi-polar solvents are here defined as solvents with a dielectricity constant in the range of 20-50, whereas polar and non-polar solvents are defined by a dielectricity constant above 50 and below 20, respectively. Examples of semi-polar protic are listed in Table 1 together with water as a reference.

Table 1.Dielectricity constants (static permittivity) of selected semi-polar organic protic solvents and water as a reference(Handbook of Chemistry and Physics, CMC Press, dielectricity constants are measured in static electric fields or at relatively low frequencies, where no relaxation occurs)

| Solvent (Temperature , Kelvin) | Dielectricity constant, $\epsilon$* |
| --- | --- |
| Water (293.2) | 80.1 |
| Propanetriol [Glycerol] (293.2) | 46.53 |
| Ethanediol [Ethylene Glycol] (293.2) | 41.4 |

(continued)

| Solvent (Temperature , Kelvin) | Dielectricity constant, $\varepsilon^*$ |
|---|---|
| 1,3-propanediol (293.2) | 35.1 |
| Methanol (293.2) | 33.0 |
| 1,4-butanediol (293.2) | 31.9 |
| 1,3-butanediol (293.2) | 28.8 |
| 1,2-propanediol [propylene glycol] (303.2) | 27.5 |
| Ethanol (293.2) | 25.3 |
| Isopropanol (293.2) | 20.18 |

In the present context, 1,2-propanediol and propylene glycol is used interchangeable. In the present context, propanetriol and glycerol is used interchangeably.In the present context, ethanediol and ethylene glycolis used interchangeably.

In one aspect of the invention, the solvent is selected from the group consisting of polyols. The term "polyol" as used herein refers to chemical compounds containing multiple hydroxyl groups.

In a further aspect of the invention, the solvent is selected from the group consisting of diols and triols. The term "diol" as used herein refers to chemical compounds containing twohydroxyl groups. The term "triol" as used herein refers to chemical compounds containing threehydroxyl groups.

In a further aspect of the invention, the solvent is selected from the group consisting of glycerol (propanetriol), ethanediol (ethylene glycol), 1,3-propanediol, methanol, 1,4-butanediol, 1,3-butanediol, propylene glycol (1,2-propanediol), ethanol and isopropanol, or mixtures thereof. In a further aspect of the invention, the solvent is selected from the group consisting of propylene glycol and glycerol. In a preferred aspect of the invention, the solvent is glycerol.This solvent is biocompatible at even high dosages and has a high solvent capacity for insulin peptides compounds.In another preferred aspect of the invention, the solvent is selected from the group consisting of propylene glycol and ethylene glycol. These solvents have a low viscosity, are biocompatible at moderate doses, and have very high solvent capacity for insulin peptides.

The solvents should preferably be of high purity with a low content of e.g. aldehydes, ketones and other reducing impurities in order to minimize chemicaldeterioration of the solubilized polypeptide due to e.g. Maillard reaction. Scavenger molecules like glycylglycine and ethylenediamine may be added to the formulations comprising semi-polar protic organic solvent(s) such as polyols to reduce deterioration of the polypeptide whereas antioxidants can be added to reduce the rate of formation of further reducing impurities.

In one aspect of the invention, the organic solvent is present in the pharmaceutical composition in an amount of at least 20% w/w.In a further aspect of the invention,the organic solvent is present in an amount of at least 30% w/w. In a further aspect of the invention,the organic solvent is present in an amount of at least 40% w/w. In a further aspect of the invention,the organic solvent is present in an amount of at least 50% w/w. In a further aspect of the invention,the organic solvent is present in an amount of at least 80% w/w.

In order to increase the shelf-stability of the pharmaceutical composition it has been found that the target pH advantageously is adjusted to between about 6.0 and about 9.0. In one aspect of the invention, the target pH is between about 6.0 and about 9.0,such as between about 6.2 and about 8.4, between about 6.4 and about 8.7, between about 6.5 and about 8.5, between about 7.0 and about 8.5, or between about 7.2 and about 8.3. In one aspect the target pH is above about 7.4, above about 7.6, above about 7.8, above about 8.0, above about 8.2, above about 8.4 or above about 8.6. It is believed that the increased shelf stability is due to fewer tendencies of the polypeptides to fibrillate after having been dehydrated as described above.

The term "shelf-stable pharmaceutical composition" as used herein means a pharmaceutical composition which is stable for at least the period which is required by regulatory agencies in connection with therapeutic proteins. Preferably, a shelf-stable pharmaceutical composition is stable for at least one year at 5 °C. Shelf-stability includes chemical stability as well as physical stability.Chemical instability involves degradation of covalent bonds, such as hydrolysis, racemization, oxidation or crosslinking. Chemical stability of the formulations is evaluated by means of reverse phase (RP-HPLC) and size exclusion chromatography (SE-HPLC).In one aspect of the invention, the formation of peptide related impurities during shelf-life is less than 10 % of the total peptide content. In a further aspect of the invention, the formation of peptide related during impurities during shelf-life is less than 5 %.The RP-HPLC analysis is typically conducted in water-acetonitrile or water-ethanol mixtures. In one embodiment, the solvent in the RP-HPLC step will comprise a salt such as $Na_2SO_4$, $(NH_4)_2SO_4$, NaCl, KCl, and buffer systems such as phosphate, and citrate and maleic acid. The required concentration of salt in the solvent may be from about 0.1 M to about 1 M, preferable between 0.2 M to 0.5 M, most preferable between 0.3 to 0.4 M. Increase of the concentration of salt requires an increase in the concentration of organic solvent in order to achieve elution from the column within a suitable time.

Physical instability involves conformational changes relative to the native structure, which includes loss of higher order structure, aggregation, fibrillation, precipitation or adsorption to surfaces. For example insulin peptidesand amylin compounds are known to be prone to instability due to fibrillation.Physical stability of the formulations may be evaluated by conventional means of e.g. visual inspection, nephelometry and Thioflavin T assay after storage of the formulation at different temperatures for various time periods.

Conformational stability can be evaluated by circular dichroism and NMR as described by e.g. Hudson and Andersen, Peptide Science, vol 76 (4), pp. 298-308 (2004).

The term "therapeutically active polypeptide" or "therapeutic polypeptides" as used herein refers to a polypeptide able to cure, alleviate or partially arrest the clinical manifestations of a given disease and its complications.

In a further aspect of the invention, the term "therapeutically active polypeptide" or "therapeutic polypeptides" as used herein means a polypeptide which is being developed for therapeutic use, or which has been developed for therapeutic use.

An amount adequate to accomplish this is defined as "therapeutically effective amount".

Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

The term "polypeptide" or "peptide" isused interchangeably herein to mean a compound composed of at least five constituent amino acids connected by peptide bonds. The constituent amino acids may be from the group of the amino acids encoded by the genetic code and they may be natural amino acids which are not encoded by the genetic code, as well as synthetic amino acids. Natural amino acids which are not encoded by the genetic code are e.g. hydroxyproline, γ-carboxyglutamate, ornithine, phosphoserine, D-alanine and D-glutamine. Synthetic amino acids comprise amino acids manufactured by chemical synthesis, i.e. D-isomers of the amino acids encoded by the genetic code such as D-alanine and D-leucine, Aib (α-aminoisobutyric acid), Abu (α-aminobutyric acid), Tle (tert-butylglycine), β-alanine, 3-aminomethyl benzoic acid, anthranilic acid.

The production of polypeptides and peptides is well known in the art. Polypeptides or peptides may for instance be produced by classical peptide synthesis, e.g. solid phase peptide synthesis using t-Boc or Fmoc chemistry or other well established techniques, see e.g. Greene and Wuts, "Protective Groups in Organic Synthesis", John Wiley & Sons, 1999. The polypeptides or peptides may also be produced by a method which comprises culturing a host cell containing a DNA sequence encoding the (poly)peptide and capable of expressing the (poly)peptide in a suitable nutrient medium under conditions permitting the expression of the peptide. For (poly)peptides comprising non-natural amino acid residues, the recombinant cell should be modified such that the non-natural amino acids are incorporated into the (poly)peptide, for instance by use of tRNA mutants.

The term "pharmaceutical composition" as used herein means a product comprising a therapeutically active polypeptide together with pharmaceutical excipients such as, a surfactant, buffer, preservative and tonicity modifier, said pharmaceutical composition being useful for treating, preventing or reducing the severity of a disease or disorder by administration of said pharmaceutical composition to a person. Thus a pharmaceutical composition is also known in the art as a pharmaceutical formulation. It is to be understood that pH of a pharmaceutical composition which is to be reconstituted is the pH value which is measured on the reconstituted composition produced by reconstitution in the prescribed reconstitution liquid at room temperature.

The term "pharmaceutically acceptable" as used herein means suited for normal pharmaceutical applications, i.e. giving rise to no serious adverse events in patients etc.

The term "buffer" as used herein refers to a chemical compound in a pharmaceutical composition that reduces the tendency of pH of the composition to change over time as would otherwise occur due to chemical reactions. Buffers include chemicals such as sodium phosphate, TRIS, glycine and sodium citrate.

The term "preservative" as used herein refers to a chemical compound which is added to a pharmaceutical composition to prevent or delay microbial activity (growth and metabolism). Examples of pharmaceutically acceptable preservatives are phenol, m-cresol and a mixture of phenol and m-cresol.

The term "stabilizer" as used herein refers to chemicals added to peptide containing pharmaceutical compositions in order to stabilize the peptide, i.e. to increase the shelf life and/or in-usetime of such compositions. Examples of stabilizers used in pharmaceutical formulations are L-glycine, L-histidine, arginine, glycylglycine, ethylenediamine, citrate, EDTA,polyethylene glycol, carboxymethylcellulose, and surfactants and antioxidants like alfa-tocopherol and l-ascorbic acid.

The term "surfactant" as used herein refers to any substance, in particular a detergent, that can adsorb at surfaces and interfaces, like liquid to air, liquid to liquid, liquid to container or liquid to any solid. The surfactant may be selected from a detergent, such asethoxylated castor oil, polyglycolyzed glycerides, acetylated monoglycerides, sorbitan fatty acid esters, polysorbate, such as polysorbate-20, poloxamers, such as poloxamer 188 and poloxamer 407, polyoxyethylenesorbitan fatty acid esters, polyoxyethylene derivatives such as alkylated and alkoxylated derivatives (tweens, e.g. Tween-20, or Tween-80), monoglycerides or ethoxylated derivatives thereof, diglycerides or polyoxyethylene derivatives thereof, glycerol, cholic acid or derivatives thereof, lecithins, alcohols and phospholipids, glycerophospholipids (lecithins, kephalins, phosphatidyl serine), glyceroglycolipids (galactopyransoide), sphingophospholipids (sphingomyelin), and sphingoglycolipids (ceramides, gangliosides), DSS (docusate sodium, CAS registry no [577-11-7]), docusate calcium, CAS registry no [128-49-4]), docusate potassium, CAS registry no [7491-09-0]), SDS (sodium dodecyl sulfate or sodium lauryl sulfate), dipalmitoylphosphatidic acid, sodium caprylate, bile acids and salts thereof and glycine or taurine conjugates, ursodeoxycholic acid, sodium cholate, sodium deoxycholate, sodium taurocholate, sodium glycocholate, N-Hexadecyl-N,N-dimethyl-3-ammonio-1-propanesulfonate, anionic (alkyl-aryl-sulphonates) monovalent surfactants, palmitoyllysophosphatidyl-L-serine, lysophospholipids (e.g. 1-acyl-sn-glycero-3-phosphate esters of ethanolamine, choline, serine or threonine), alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the postively charged DODAC, DOTMA, DCP, BISHOP, lysophosphatidylserine and lysophosphatidylthreonine, zwitterionic surfactants (e.g. N-alkyl-N,N-dimethylammonio-1-propanesulfonates, 3-cholamido-1-propyldimethylammonio-1-propanesulfonate, dodecylphosphocholine, myristoyllysophosphatidylcholine, hen egg lysolecithin), cationic surfactants(quarternary ammonium bases) (e.g. cetyl-trimethylammonium bromide, cetylpyridinium chloride), non-ionic surfactants (e. g. alkyl glucosides like dodecyl β-D-glucopyranoside, dodecyl β-D-maltoside, tetradecylβ-D-glucopyranoside, decylp-D-maltoside,dodecyl β-D-maltoside, tetradecylβ-D-maltoside, hexadecylβ-D-maltoside, decylβ-D-maltotrioside,dodecyl β-D-maltotrioside, tetradecylβ-D-maltotrioside, hexadecylβ-D-maltotrioside, n-dodecyl-sucrose, n-decyl-sucrose, fatty alcohol ethoxylates (e. g. polyoxyethylene alkyl ethers like octaethylene glycol mono tridecyl ether, octaethylene glycol mono dodecyl ether,octaethylene glycol mono tetradecyl ether), block copolymers as polyethyleneoxide/polypropyleneoxide block copolymers (Pluronics/Tetronics, Triton X-100) ethoxylatedsorbitanalkanoates surfactants (e. g. Tween-40, Tween-80, Brij-35), fusidic acid derivatives (e.g. sodium tauro-dihydrofusidate etc.), long-chain fatty acids and salts thereof C6-C12 (eg. oleic acid and caprylic acid), acylcarnitines and derivatives, $N^\alpha$-acylated derivatives of lysine, arginine or histidine, or side-chain acylated derivatives of lysine or arginine, $N^\alpha$-acylated derivatives of dipeptides comprising any combination of lysine, arginine or histidine and a neutral or acidic amino acid, $N^\alpha$-acylated derivative of a tripeptide comprising any combination of a neutral amino acid and two charged amino acids, or the surfactant may be selected from the group of imidazoline derivatives, or mixtures thereof.

The term "treatment of a disease" as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder, and prevention of the disease, condition or disorder.

The term "prevention of a disease" as used herein is defined as the management and care of an individual at risk of developing the disease prior to the clinical onset of the disease. The purpose of prevention is to combat the development of the disease, condition or disorder, and includes the administration of the active compounds to prevent or delay the onset of the symptoms or complications and to prevent or delay the development of related diseases, conditions or disorders.

The term "analogue" as used herein referring to a peptide means a modified peptide wherein one or more amino acid residues of the peptide have been substituted by other amino acid residues and/or wherein one or more amino acid residues have been deleted from the peptide and/or wherein one or more amino acid residues have been added to the peptide. Such addition or deletion of amino acid residues can take place at the N-terminal of the peptide and/or at the C-terminal of the peptide. In one embodiment an analogue comprises less than 6 modifications (substitutions, deletions, additions) relative to the native peptide. In another embodiment an analogue comprises less than 5 modifications (substitutions, deletions, additions) relative to the native peptide. In another embodiment an analogue comprises less than 4 modifications (substitutions, deletions, additions) relative to the native peptide. In another embodiment an analogue comprises less than 3 modifications (substitutions, deletions, additions) relative to the native peptide. In another embodiment an analogue comprises less than 2 modifications (substitutions, deletions, additions) relative to the native peptide. In another embodiment an analogue comprises only a single modification (substitutions, deletions, additions) relative to the native peptide. The added and/or exchanged amino acid residues can either be codable amino acid residues or other naturally occurring residues or purely synthetic amino acid residues.

The term "derivative" as used herein in relation to a parent peptide means a chemically modified parent protein or an analogue thereof, wherein at least one substituent is not present in the parent protein or an analogue thereof, i.e. a parent protein which has been covalently modified. Typical modifications are amides, carbohydrates, alkyl groups, acyl groups, esters, PEGylations and the like.

The term "GLP-1 compound" as used herein means GLP-1(7-37) (SEQ ID NO. 1), insulinotropic analogue thereof and insulinotropic derivatives thereof.

The term "insulinotropic" as used herein referring to a peptide or a compound means the ability to stimulate secretion of insulin in response to an increased plasma glucose level. Insulinotropic peptides and compounds are agonists of the GLP-1 receptor. The insulinotropic property of a compound may be determined by in vitro or in vivo assays known in the art.

The term "exendin-4 compound" as used herein is defined as exendin-4(1-39) (SEQ ID NO. 2), insulinotropic fragments thereof, insulinotropicanalogs thereof and insulinotropic derivatives thereof.

With "insulin peptide" as used herein is meant human insulin, porcine insulin or bovine insulin with disulfide bridges between CysA7 and CysB7 and between CysA20 and CysB19 and an internal disulfide bridge between CysA6 and CysA11 or an insulin analogue or derivative thereof.

An insulin analogue as used herein is a polypeptide which has a molecular structure which formally can be derived from the structure of a naturally occurring insulin, for example that of human insulin, by deleting and/or substituting at least one amino acid residue occurring in the natural insulin and/or by adding at least one amino acid residue.

In one aspect an insulin analogue according to the invention comprises less than 8 modifications (substitutions, deletions, additions) relative to the parent insulin. In one aspect an insulin analogue comprises less than 7 modifications (substitutions, deletions, additions) relative to the parent insulin. In one aspect an insulin analogue comprises less than 6 modifications (substitutions, deletions, additions) relative to the parent insulin. In another aspect an insulin analogue comprises less than 5 modifications (substitutions, deletions, additions) relative to the parent insulin. In another aspect an insulin analogue comprises less than 4 modifications (substitutions, deletions, additions) relative to the parent insulin. In another aspect an insulin analogue comprises less than 3 modifications (substitutions, deletions, additions) relative to the parent insulin. In another aspect an insulin analogue comprises less than 2 modifications (substitutions, deletions, additions) relative to the parent insulin.

The insulin analogues may be such that e.g. a fast onset of action, a protracted action and/or a stability towards proteases is obtained.

In one aspect the insulin analogues are such that a fast onset of action is obtained, i.e. the onset of action of the insulin analogue is within 4 hours, alternatively 3 hours, 2 hours, 1 hour or ½ hour after administration. In another aspect the insulin analogues are such that a protracted action is obtained, i.e. the action of the insulin analogue is continued for more than 4 hours, alternatively 6 hours, 8 hours, 12 hours, 18 hours or 24 hours after administration.

The insulin analogues may be such wherein position 28 of the B chain may be modified from the natural Pro residue to one of Asp, Lys, Leu, Val, Ala or Ile. In another aspect Lys at position B29 of insulin is modified to Pro or Glu.In one aspect an insulin analogue according to the invention is such wherein the amino acid residue in position B28 of insulin is Pro, Asp, Lys, Leu, Val, or Ala and the amino acid residue in position B29 is Lys or Pro and optionally the amino acid residue in position B30 is deleted. Also, Asn at position A21 may be modified to Ala, Gln, Glu, Gly, His, Ile, Leu, Met, Ser, Thr, Trp, Tyr or Val, in particular to Gly, Ala, Ser, or Thr and preferably to Gly. Furthermore, Asn at position B3 may be modified to Lys,Thr, Ser, Gln, Glu or Asp. Further examples of insulin analogues are des(B30) human insulin; des(B30) human insulin analogues; insulin analogues wherein PheB1 has been deleted; insulin analogues wherein the A-chain and/or the B-chain have an N-terminal extension and insulin analogues wherein the A-chain and/or the B-chain have a C-terminal extension. Thus one or two Arg may be added to position B1.In another aspect an insulin analogue according to the invention is des(B28-B30) human insulin, des(B27) human insulin or des(B30) human insulin. In yet another aspect an insulin analogue according to the invention is an insulin analogue wherein the amino acid residue in position B3 is Lys and the amino acid residue in position B29 is Glu or Asp.

In another aspect an insulin analogue according to the invention is des(B28-B30) human insulin, des(B27) human insulin or des(B30) human insulin. In yet another aspect an insulin analogue according to the invention is an insulin analogue wherein the amino acid residue in position B3 is Lys and the amino acid residue in position B29 is Glu or Asp.

Insulin analogues according to the invention may beproteolytically stableinsulin analogues, i.e. protected towards degradation by proteases. A non-limiting example of a proteolytically stableinsulin analogues is e.g. described in WO 2008/034881 (Novo Nordisk).

In one aspect an insulin analogue according to the invention isa proteolytically stableinsulin analogue.

By "a proteolytically stableinsulin analogue" as used herein is meant an insulin analogue which comprises one or more mutations, i.e. one or more substitutions, additions, insertions and/or deletions relative to human insulin, and which is subjected to slower degradation by one or more proteases relative to human insulin. In one aspect of the invention an insulin analogue according to the invention is stabilized against degradation by one or more enzymes selected from the group consisting of: pepsin (such as e.g. the isoforms pepsin A, pepsin B, pepsin C and/or pepsin F), chymotrypsin (such as e.g. the isoforms chymotrypsin A, chymotrypsin B and/or chymotrypsin C), trypsin, Insulin-Degrading Enzyme (IDE), elastase (such as e.g. the isoforms pancreatic elastase I and/or II), carboxypeptidase (e.g. the isoforms carboxypeptidase A, carboxypeptidase A2 and/or carboxypeptidase B), aminopeptidase, cathepsin D and other enzymes present in intestinal extracts derived from rat, pig or human.

In one aspect of the invention a proteolytically stableinsulin analogueis an insulin analoguewherein the amino acid in position A14 is Glu or His, the amino acid in position B25 is His and which optionally further comprises one or more additional mutations; an insulin analogue wherein

> • the amino acid in position A8 is His and/or the amino acid in position A12 is Glu or Asp and/or the amino acid in position A13 is His, Asn, Glu or Asp and/or the amino acid in position A14 is Asn, Gln, Glu, Arg, Asp, Gly or His and/or the amino acid in position A15 is Glu or Asp; and
> • the amino acid in position B1 is Glu and/or the amino acid in position B16 is Glu or His and or the amino acid in position B25 is His and/or the amino acid in position B26 is His, Gly, Asp or Thr and/or the amino acid in position B27 is His, Glu, Lys, Gly or Arg and/or the amino acid in position B28 is His, Gly or Asp; and

which optionally further comprises one or more additional mutations; or an insulin analogue wherein the amino acid in position A14 isselected from the group consisting of Lys, Glu, Arg, Asp, Pro and His; and the B-chain of the insulin analogue comprises at least two mutations relative to the parent insulin, wherein two or more mutations are in the form of deletions of the amino acids in positions B27, B28, B29 and B30, or a combination of a deletion of the amino acid in position B30 and a substitution of an amino acid selected from the amino acid substitutions in position: B25 to His, B26 to Gly or Glu, B27 to Gly or Lys and B28 to Asp, His, Gly, Lys or Glu.

In a yet further aspect an insulin of the invention is selected from the group consisting of: human insulin; DesB30 human insulin; AspB28 human insulin; AspB28,DesB30 human insulin; LysB3,GluB29 human insulin; LysB28,ProB29 human insulin; GluA14,HisB25 human insulin; HisA14,HisB25 human insulin; GluA14,HisB25,DesB30 human insulin; HisA14, HisB25,DesB30 human insulin; GluA14,HisB25,desB27,desB28,desB29,desB30 human insulin; GluA14,HisB25,GluB27,desB30 human insulin; GluA14,HisB16,HisB25,desB30 human insulin; HisA14,HisB16,HisB25,desB30 human insulin; HisA8,GluA14,HisB25,GluB27,desB30 human insulin; HisA8,GluA14,GluB1,GluB16,HisB25,GluB27,desB30 human insulin; and HisA8,GluA14,GluB16,HisB25,desB30 human insulin.

With "desB30 insulin", "desB30 human insulin"is meant insulin or an analogue thereof lacking the B30 amino acid residue.

By "parent insulin" is meant a naturally occurring insulin such as human insulin or porcine insulin. Alternatively, the parent insulin can be an insulin analogue.

In one aspect of the present invention, the therapeutically active polypeptide is an insulin peptide.

In one aspect of the invention, the insulin peptide is human insulin, an analog of human insulin, a derivative of human insulin or a derivative of a human insulin analog.

In one aspect of the invention, the insulin peptide is human insulin.

In one aspect of the invention, the insulin peptide is an insulin derivative. In a further aspect of the invention, the insulin derivative is selected from the group consisting of B29-N$^\epsilon$-myristoyl-des(B30) human insulin, B29-N$^\epsilon$-palmitoyl-des(B30) human insulin, B29-N$^\epsilon$-myristoyl human insulin, B29-N$^\epsilon$-palmitoyl human insulin, B28-N$^\epsilon$-myristoyl Lys$^{B28}$ Pro$^{B29}$ human insulin, B28-N$^\epsilon$-palmitoyl Lys$^{B28}$ Pro$^{B29}$ human insulin, B30-N$^\epsilon$-myristoyl-Thr$^{B29}$Lys$^{B30}$ human insulin, B30-N$^\epsilon$-palmitoyl-Thr$^{B29}$Lys$^{B30}$ human insulin, B29-N$^\epsilon$-(N-palmitoyl-γ-glutamyl)-des(B30) human insulin, B29-N$^\epsilon$-(N-lithocholyl-y-glutamyl)-des(B30) human insulin, B29-N$^\epsilon$-(ω-carboxyheptadecanoyl)-des(B30) human insulin and B29-N$^\epsilon$-(ω-carboxyheptadecanoyl) human insulin.

In another aspect of the invention, the insulin derivative is B29-N$^\epsilon$-myristoyl-des(B30) human insulin.

In a further aspect of the invention the insulin peptide is an acid-stabilised insulin.

The acid-stabilised insulin may be selected from analogues of human insulin having one of the following amino acid residue substitutions:

A21G
A21G, B28K, B29P
A21G, B28D
A21G, B28E
A21G, B3K, B29E
A21G, desB27
A21G, B9E
A21G, B9D
A21G, B10E.

In a further aspect of the invention, the insulin peptide is an insulin analogue. The insulin analogue may be selected from the group consisting of an analogue wherein position B28 is Asp, Lys, Leu, Val, or Ala and position B29 is Lys or Pro; and des(B28-B30), des(B27) or des(B30) human insulin.

In another aspect of the invention, the insulin analogue is an analogue of human insulin wherein position B28 is Asp or Lys, and position B29 is Lys or Pro.

In another aspect of the invention, the insulin analogue is des(B30) human insulin.

In another aspect of the invention, the insulin analogue is an analogue of human insulin wherein position B28 is Asp.

In another aspect of the invention, the insulin analogue is an analogue wherein position B3 is Lys and position B29 is Glu or Asp.

In another aspect of the invention, the insulin analogs and derivatives are selected from among those disclosed in EP 0 792 290 (Novo Nordisk A/S), EP 0 214 826 and EP 0 705 275 (Novo Nordisk A/S), US 5,504,188 (Eli Lilly), EP 0 368 187 (Aventis), US patents 5,750,497 and 6,011,007, EP 375437 and EP 383472 and where such insulins may include, but are not limited to, insulin glulisine ( also known as Apidra®, differs from human insulin in that the amino acid asparagine at position B3 is replaced by lysine and the lysine in position B29 is replaced by glutamic acid), Lys$^{B28}$ Pro$^{B29}$ human insulin (Humalog®) , and Asp$^{B28}$ human insulin (insulin aspart (Novolog®)).

In one aspect of the invention, said human insulin analog is Asp$^{B28}$-human insulin. In another aspect of the invention, said human insulin analog is Lys$^{B28}$,Pro$^{B29}$-human insulin. In another aspect of the invention, said human insulin analog is Lys$^{B3}$,Glu$^{B29}$-human insulin (insulin glulisine). In another aspect of the invention, said human insulin analog is des(B30) human insulin.

Also, derivatives of precursors or intermediates are covered by the invention. An example of such a derivative is a single-chain insulin which comprises the B- and the A-chain of human insulin or analogues or derivatives thereof connected by a connecting peptide.

An insulin derivative according to the invention is a naturally occurring insulin or an insulin analogue which has been chemically modified, e.g. by introducing a side chain in one or more positions of the insulin backbone or by oxidizing or reducing groups of the amino acid residues in the insulin or by converting a free carboxylic group to an ester group or to an amide group. Other derivatives are obtained by acylating a free amino group or a hydroxy group, such as in the B29 position of human insulin or desB30 human insulin. A non-limiting example of acylated polypeptides may e.g. be found in WO 95/07931 which is are hereby incorporated by reference.

In one aspect of the invention, the therapeutically active polypeptide has a molar weight of less than 100 kDa, less than 50 kDa, or less than 10 kDa.

In one aspect of the invention, the therapeutically active polypeptide has a molar weight of less than 100 kDa, less than 50 kDa, or less than 10 kDa, with the proviso that the polypeptide is not insulinotropic peptide, GLP-1(7-37) or an analog or derivative thereof, or exendin or an analog or derivative thereof.

In another aspect of the invention, the therapeutically active polypeptide comprises less than 100 amino acids, or less than 90 amino acids, or less than 60 amino acids. In another aspect of the invention, the therapeutically active polypeptide comprises at least 10 amino acids, at least 15 amino acids, or at least 20 amino acids. In a further aspect of the invention, the therapeutically active polypeptide comprises 10 - 100 amino acids, in a further aspect 15 - 90 amino acids, in a further aspect 20-80 amino acids, in a further aspect 20 - 70 amino acids, in a further aspect 25 - 70 amino acids, in yet a further aspect 25 - 65 amino acids, in yet a further aspect 25 - 60 amino acids or 25 - 55 amino acids. In a yet further aspect,the therapeutically active polypeptide comprises 30 - 70 amino acids, 30 - 65 amino acids, 30 - 60 amino acids or 30 - 55 amino acids.

In another aspect of the invention, the therapeutically active polypeptide comprises less than 100 amino acids, or less than 90 amino acids, or less than 60 amino acids. In another aspect of the invention, the therapeutically active polypeptide comprises at least 10 amino acids, at least 15 amino acids, or at least 20 amino acids. In a further aspect of the invention, the therapeutically active polypeptide comprises 10 - 100 amino acids, in a further aspect 15 - 90 amino acids, in a further aspect 20-80 amino acids, in a further aspect 20 - 70 amino acids, in a further aspect 25 - 70 amino acids, in yet a further aspect 25 - 65 amino acids, in yet a further aspect 25 - 60 amino acids or 25 - 55 amino acids. In a yet further aspect,the therapeutically active polypeptide comprises 30 - 70 amino acids, 30 - 65 amino acids, 30 - 60 amino acids or 30 - 55 amino acids, with the proviso that the polypeptide is not insulinotropic peptide, GLP-1(7-37) or an analog or derivative thereof, or exendin or an analog or derivative thereof.

In one aspect of the invention, the therapeutically active polypeptide is a polypeptide which is water soluble. In another aspect, the therapeutically active polypeptide is water soluble at a concentration of at least 100 μg polypeptide per ml solution at 25°C. In yet another aspect, the therapeutically active polypeptide is watersoluble at pH values which are at least 2 pH units from the isoelectric point of the polypeptide in aqueous solution.Thus, in one aspect of the invention, the polypeptide is water soluble at pH values more than 2 pH units above the isoelectric point of the polypeptide. In another aspect of the invention, the polypeptide is water soluble at pH values more than 2 pH units below the isoelectric point of the polypeptide. In a further aspect, the polypeptide is water soluble at pH values more than 2.5 pH units above or below the pI of the polypeptide. In yet a further aspect, the polypeptide is water soluble at pH values 3.0 pH units above or below the pI of the polypeptide. In a further aspect, the polypeptide is water soluble at pH values 3.5 pH units above or below the pI of the polypeptide.

In one aspect of the invention, the therapeutically active polypeptide is a polypeptide which is water soluble according to any of the above aspects, with the proviso that the polypeptide is not insulinotropic peptide, GLP-1(7-37) or an analog or derivative thereof, or exendin or an analog or derivative thereof.

By "water soluble" is meant that a large concentration polypeptide, such as 100 μg polypeptide per ml solution, dissolves in an aqueous or buffer solution at 25°C. Methods for determining whether the polypeptide contained in a solution is dissolved are known in the art.

In one embodiment, the solution may be subjected to centrifugation for 20 minutes at 30,000 g and then the polypeptide concentration in the supernatant may be determined by RP-HPLC. If this concentration is equal within experimental error to the polypeptide concentration originally used to make the composition, then the polypeptide is fully soluble in the composition of the invention.

In another embodiment, the solubility of the polypeptide in a composition of the invention can simply be determined by examining by eye the container in which the composition is contained. The polypeptide is soluble if the solution is clear to the eye and no particulate matter is either suspended or precipitated on the sides/bottom of the container.

It is known to the skilled person that e.g. cyclosporines are not water soluble.

In anaspect of the invention, the therapeutically active polypeptide is selected from the group consisting of single chain insulin (such as e.g. described in WO 2005/054291), insulin mimetics (such as e.g. described in WO 2006/018450), a polypeptide that binds to the MC4 receptor, human growth hormone or an analog thereof, factor VII or an analog thereof, parathyroid hormone or an analog thereof, human follicle stimulating hormone or an analog thereof, a growth factor such as platelet-derived growth factor (PDGF), Obestatin, transforming growth factor α (TGF-α), transforming growth factor β (TGF-β), epidermal growth factor (EGF), vascular endothelial growth factor (VEGF), a somatomedin such as insulin growth factor I (IGF-I), insulin growth factor II (IFG-II), erythropoietin (EPO), thrombopoietin (TPO) or angiopoietin, interferon, pro-urokinase, urokinase, tissue plasminogen activator (t-PA), plasminogen activator inhibitor 1, plasminogen activator inhibitor 2, von Willebrandt factor, a cytokine, e.g. an interleukin such as interleukin (IL) 1, IL-1Ra, IL-2, IL-4, IL-5, IL-6, IL-9, IL-11, IL-12, IL-13, IL-15, IL-16, IL-17, IL-18, IL-20 or IL-21, a colony stimulating factor (CFS) such as GM-CSF, stem cell factor, a tumor necrosis factor such as TNF-α, lymphotoxin-α, lymphotoxin-β, CD40L, or CD30L, a protease inhibitor e.g. aprotinin, an enzyme such as superoxide dismutase, asparaginase, arginase, arginine deaminase, adenosine deaminase, ribonuclease, catalase, uricase, bilirubin oxidase, trypsin, papain, alkaline phosphatase, β-glucoronidase, purine nucleoside phosphorylase or batroxobin, an opioid, e.g. endorphins, enkephalins or non-natural opioids, a hormone or neuropeptide, e.g. calcitonin, glucagon, gastrins, adrenocorticotropic hormone (ACTH), cholecystokinins, lutenizing hormone, gonadotropin-releassing hormone, chorionic gonadotropin, corticotrophin-releasing factor, vasopressin, oxytocin, antidiuretic hormones, thyroid-stimulating hormone, thyrotropin-releasing hormone, relaxin, prolactin, peptide YY, neuropeptide Y, pancreastic polypeptide, leptin, CART (cocaine and amphetamine regulated transcript), a CART related peptide, perilipin, peptide hormones acting on the melanocortin receptors such as α-MSH or ACTH, melanin-concentrating hormones, natriuretic peptides, adrenomedullin, endothelin, secretin, amylin, vasoactive intestinal peptide (VIP), pituaryadenylatecyclase activating polypeptide (PACAP), bombesin, bombesin-like peptides, thymosin, heparin-binding protein, soluble CD4, hypothalmic releasing factor, melanotonins and analogs thereof.

Conformational stability protein based drugs is important for maintaining biological activity and for minimizing irreversible loss of structure due to denaturation and fibrillation. Especially large polypeptides and proteins are labile with respect to conformational change due to complicated refolding patterns. Also, polypeptides with a known history of fibrillation, such as insulin and amylin, are particularly sensitive towards destabilization of tertiary structure (i.e. formation of a molten globular state).

Effective amounts for each purpose will depend on the severity of the disease or injury as well as the weight and general state of the subject.

In one aspect of the invention, the pharmaceutical formulation comprises a therapeutically active polypeptide in a concentration from 0.1 % w/w to 50 % w/w.

Effective amounts for each purpose will depend on the severity of the disease or injury as well as the weight and general state of the subject.

The term "about" as used herein means in reasonable vicinity of the stated numerical value, such as plus or minus 10%.

The present invention further provides a process for the preparation of a pharmaceutical solution by:

a) providing an aqueous solution of a therapeutically active polypeptideoptionally comprising excipients,

b) adjusting the pH value to a target pH value which is 1 unit, preferably 2 units and more preferably 2.5 pH units above or below the pI of the polypeptide,

c) removing water (dehydrating) the polypeptide by conventional drying technologies such as freeze- and spray drying, and

d) mixing and dissolution of the polypeptide in a semi-polar protic non-aqueous solvent e.g. by stirring, tumbling or other mixing methods,

e) optionally filtration or centrifugation of the non-aqueous polypeptide solution to remove non-dissolved inorganic salts,

f) optionally removing residual amounts of waters by e.g. adding solid dessicants or vacuum drying,

g) optionally adding further excipients such as a hydrofluoroalkane propellants and cosolvents for solution pressurized metered dose inhalers or adding detergents, polymers, lipids and co-solvents for oral dosage forms.

The present invention further provides a process for the preparation of a pharmaceutical solution by:

a) providing an aqueous solution of a therapeutically active polypeptideoptionally comprising excipients,

b) adjusting the pH value to a target pH value which is 1 unit, preferably 2 units and more preferably 2.5 pH units above or below the pI of the polypeptide,

c) removing water (dehydrating) the polypeptide by conventional drying technologies such as freeze- and spray drying, and

d) mixing and dissolution of the polypeptide in a semi-polar protic non-aqueous solvent e.g. by stirring, tumbling or other mixing methods,

e) optionally filtration or centrifugation of the non-aqueous polypeptide solution to remove non-dissolved inorganic salts,

f) optionally removing residual amounts of waters by e.g. adding solid dessicants or vacuum drying,

g) optionally adding further excipients such as a hydrofluoroalkane propellants and cosolvents for solution pressurized metered dose inhalers or adding detergents, polymers, lipids and co-solvents for oral dosage forms,

with the proviso that the polypeptide is not insulinotropic peptide, GLP-1(7-37) or an analog or derivative thereof, or exendin or an analog or derivative thereof.

The present invention further provides a process for the preparation of a pharmaceutical composition by:

a) providing an aqueous solution of a therapeutically active polypeptide, optionally containing stabilizers such as zinc and glycylglycine,

b) adjusting the pH value to 1 unit, preferably 2 units and more preferably 2.5 pH units above or below the pI of the polypeptide e.g. by adding a non-volatile base or a acid, such as hydrochloric acid or sodium hydroxide, to the solution

c) removing water (dehydrating) the polypeptide by conventional drying technologies such as freeze- and spray drying,

d) mixing and dissolution of the polypeptide in a semi-polar protic non-aqueous solvent e.g. by stirring, tumbling or other mixing methods,

e) optionally filtration or centrifugation of the non-aqueous polypeptide solution to remove non-dissolved inorganic salts,

f) optionally removing residual amounts of waters by e.g. adding solid dessicants or vacuum drying,

g) optionally adding further excipients such as a hydrofluoroalkane propellants and cosolvents for solution pressurized metered dose inhalers or adding detergents, polymers, lipids and co-solvents for oral dosage forms.

The present invention further provides a process for the preparation of a pharmaceutical composition by:

a) providing an aqueous solution of a therapeutically active polypeptide, optionally containing stabilizers such as zinc and glycylglycine,

b) adjusting the pH value to 1 unit, preferably 2 units and more preferably 2.5 pH units above or below the pI of the polypeptide e.g. by adding a non-volatile base or a acid, such as hydrochloric acid or sodium hydroxide, to the solution

c) removing water (dehydrating) the polypeptide by conventional drying technologies such as freeze- and spray drying,

d) mixing and dissolution of the polypeptide in a semi-polar protic non-aqueous solvent e.g. by stirring, tumbling or other mixing methods,

e) optionally filtration or centrifugation of the non-aqueous polypeptide solution to remove non-dissolved inorganic salts,

f) optionally removing residual amounts of waters by e.g. adding solid dessicants or vacuum drying,

g) optionally adding further excipients such as a hydrofluoroalkane propellants and cosolvents for solution pressurized metered dose inhalers or adding detergents, polymers, lipids and co-solvents for oral dosage forms,

with the proviso that the polypeptide is not insulinotropic peptide, GLP-1(7-37) or an analog or derivative thereof, or exendin or an analog or derivative thereof.

In one aspect of the invention, the polypeptide is added to an aqueous solution. The aqueous solution can be pure water or it can contain excipients or an alkaline solution. In one aspect of the invention, the pH of the polypeptide solution is adjusted with an alkaline solution comprising a non-volatile base. The non volatile base can be selected from the group consisting of alkaline metal salts, alkaline metal hydroxides, alkaline earth metal salts, alkaline earth metal hydroxides and amino acids or a combination hereof. For example the pH can be adjusted with sodium hydroxide, potassium hydroxide, calcium hydroxide, calcium oxide or any combination hereof. In another aspect of the invention, the pH of the polypeptide solution is adjusted with anon-volatile acid selected fromhydrochloric acid, phosphoric acid and sulfuric acid.

In one aspect of the invention, the polypeptide non-aqueous solution comprises glycylglycine. Glycylglycine might act as a scavenger for reductive impurities in the solvent such as e.g. glyceraldehyde. In one aspect glycylglycine is added to a propylene glycol solution comprising insulin e.g. in a concentration of glycylglycine in the solution ofbetween about 4 mM and about 200 mM.

In one aspect of the invention, the polypeptidenon-aqueoussolution comprises Tween 80 and oleic acid. When Tween 80 and oleic acid is added to a non-aqueouspolypeptide solution, the polypeptide solutionforms a microemulsion upon dilution with an aqueous liquid following oral administration. The microemulsion might act as an absorption enhancer and furthermore generate more reproducible absorption kinetics. The concentration of Tween 80 in the solution will e.g. be between about 30 and 70 % w/w and the concentration of oleic acid will e.g. be between 10 and 30 % w/w.

In one aspect, the invention is related to a pharmaceutical composition for the treatment of type 1 diabetes, type 2 diabetes and other states that cause hyperglycaemia in patients in need of such a treatment.

In one aspect, the invention is related to a pharmaceutical composition according to the invention with a pharmaceutically acceptable carrier and/or a pharmaceutically acceptable additive, which composition can be provided for the treatment of type 1 diabetes, type 2 diabetes and other states that cause hyperglycaemia in patients in need of such a treatment.

In one aspect of the invention, there is provided a method of treating type 1 diabetes, type 2 diabetes and other states that cause hyperglycaemia in a patient in need of such a treatment, comprising administering to the patient a therapeutically effective amount of a pharmaceutical composition according to the invention.

In one aspect of the invention, there is provided a method of treating type 1 diabetes, type 2 diabetes and other states that cause hyperglycaemia in a patient in need of such a treatment, comprising administering to the patient a therapeutically effective amount of an pharmaceutical composition according to the invention, optionally together with a pharmaceutically acceptable carrier and/or pharmaceutical acceptable additives.

In one aspect of the invention, there is provided a method for the manufacture of a pharmaceutical composition according to the invention for the use in the treatment of type 1 diabetes, type 2 diabetes and other states that cause hyperglycaemia.

In one aspect of the invention, there is provided a method for the manufacture of a pharmaceutical composition for the use in the treatment of type 1 diabetes, type 2 diabetes and other states that cause hyperglycaemia.

**PHARMACEUTICAL COMPOSITIONS**

The compositions according to the invention can, for example, be administered subcutaneously, orally, nasally or pulmonary.

For subcutaneous administration, the composition according to the invention is formulated analogously with the formulation of known therapeutically active polypeptides. Furthermore, for subcutaneous administration, the compositions according to the invention are administered analogously with the administration of known therapeutically active polypeptides and, generally, the physicians are familiar with this procedure.

In one aspect of the invention where the therapeutically active peptide is an insulin peptide, the compositions according to the invention may be administered by inhalation in a dose effective manner to increase circulating insulin peptidelevels and/or to lower circulating glucose levels. Such administration can be effective for treating disorders such as diabetes or hyperglycaemia. Achieving effective doses of e.g. insulin requires administration of an inhaled dose of a composition according to the invention comprising more than about 0.5 $\mu$g/kg to about 50 $\mu$g/kg insulin. A therapeutically effective amount can be determined by a knowledgeable practitioner, who will take into account factors including insulin level, blood glucose levels, the physical condition of the patient, the patient's pulmonary status, or the like.

A composition according the invention may be delivered by inhalation to achieve rapid absorption of the therapeutically active polypeptide such as insulin. Administration by inhalation can result in pharmacokinetics comparable to subcutaneous administration of insulin. Inhalation of a composition according to the invention where the therapeutic polypeptide is an insulin peptide leads to a rapid rise in the level of circulating insulin followed by a rapid fall in blood glucose levels. Different inhalation devices typically provide similar pharmacokinetics when similar particle sizes and similar levels of lung deposition are compared.

According to the invention, a composition may be delivered by any of a variety of inhalation devices known in the art for administration of a therapeutic agent such as an insulin peptide by inhalation. These devices include metered dose inhalers, nebulizers, dry powder generators, sprayers, and the like. A composition of this invention is in one aspect delivered by a pressurized metered dose inhaler or a sprayer. There are a several desirable features of an inhalation device for administering a composition according to the invention. For example, delivery by the inhalation device is advantageously reliable, reproducible, and accurate. The inhalation device should deliver small particles, for example particles less than about 10 $\mu$m, for example about 1-5 $\mu$m, for good respirability. Some specific examples of commercially available inhalation devices suitable for the practice of this invention are Turbohaler™ (Astra), Rotahaler® (Glaxo), Diskus® (Glaxo), Spiros™ inhaler (Dura), devices marketed by Nektar, 3M Drug Delivery Systems andBespak, AERx™ (Aradigm), the Ultravent® nebulizer (Mallinckrodt), the Acorn II® nebulizer (Marquest Medical Products), the Ventolin® metered dose inhaler (Glaxo),the Spinhaler® powder inhaler (Fisons), or the like.

Pressurised metered dose inhalers (pMDIs) are well known devices for administering pharmaceutical products to the respiratory tract by inhalation. pMDIs comprise a pressure resistant aerosol canister typically filled with a product such as a drug dissolved in a liquefied propellant (solution formulations) or micronized particles suspended in a liquefied propellant (suspension formulations) where the container is fitted with a metering valve. Solution formulations offer the advantage of being homogeneous with the active ingredient and excipients completely dissolved in the propellant vehicle or its mixture with suitable co-solvents such as ethanol. Solution formulations also obviate physical stability problems associated with suspension formulations so assuring more consistent uniform dosage administration.

Hydrofluoroalkanes and in particular 1,1,1,2-tetrafluoroethane (HFA 134a) and 1,1,1,2,3,3,3-heptafluoropropane (HFA 227) and their mixtures havebeen acknowledged to be the best candidates for non-CFC propellants. Actuation of the metering valve allows a small portion of the spray product to be released whereby the pressure of the liquefied propellant carries the dissolved or micronized drug particles out of the container to the patient. The valve actuator is used to direct the aerosol spray into the patient's oropharynx. The canisters may consist of aluminium or stainless steel having the internal surface coated with an inert organic coating, fitted with a metering valve having a 63 microlitermetering chamber provided with a butyl rubber gasket as described in WO 03/078538.

As those skilled in the art will recognize, the formulation of the invention, the quantity of the formulation delivered, and the duration of administration of a single dose depend on the type of inhalation device employed. For some aerosol delivery systems, such as nebulizers, the frequency of administration and length of time for which the system is activated will depend mainly on the concentration of therapeutic polypeptidein the aerosol.

For example, shorter periods of administration can be used at higher concentrations of therapeutic polypeptide in the nebulizer solution. Devices such as metered dose inhalers can produce higher aerosol concentrations, and can be operated for shorter periods to deliver the desired amount of polypeptide. Devices such as powder inhalers deliver active agent until a given charge of agent is expelled from the device. In this type of inhaler, the amount of composition according to the invention in a given quantity of the powder determines the dose delivered in a single administration.

The particle size of a polypeptide such as insulin in the formulation delivered by the inhalation device is critical with respect to the ability of e.g. insulin to make it into the lungs, and into the lower airways or alveoli. The polypeptide in the composition of this invention can be formulated so that at least about 10% of the polypeptide delivered is deposited in the lung, for example about 10 to about 20%, or more. It is known that the maximum efficiency of pulmonary deposition for mouth breathing humans is obtained with particle sizes of about 2 μm to about 3 μm. Pulmonary deposition decreases substantially when particle sizes are above about 5 μm. Particle sizes below about 1 μmcause pulmonary deposition to decrease, and it becomes difficult to deliver particles with sufficient mass to be therapeutically effective. Thus, particles of polypeptide delivered by inhalation have a particle size less than about 10 μm, for example in the range of about 1 μm to about 5 μm. The formulation of polypeptide is selected to yield the desired particle size in the chosen inhalation device.

The polypeptidesolution may optionally be combined with pharmaceutical carriers or excipients which are suitable for respiratory and pulmonary administration. Such carriers may serve simply as bulking agents when it is desired to reduce the therapeutic polypeptide such as insulin concentration in the liquid which is being delivered to a patient, but may also serve to enhance the stability of the compositions.

Suitable materials include carbohydrates, e.g., (a) monosaccharides such as fructose, galactose, glucose, D-mannose, sorbose, and the like; disaccharides, such as lactose, trehalose, cellobiose, and the like; cyclodextrins, such as 2-hydroxypropyl-cyclodextrin; and polysaccharides, such as raffinose, maltodextrins, dextrans, and the like; (b) amino acids, such as glycine, arginine, aspartic acid, glutamic acid, cysteine, lysine, histidine, arginine and the like; (c) organic salts prepared from organic acids and bases, such as sodium citrate, sodium ascorbate, magnesium gluconate, sodium gluconate, tromethamine hydrochloride, and the like; (d) peptides and proteins, such as aspartame, human serum albumin, gelatin, and the like; (e) alditols, such as mannitol, xylitol, and the like. A preferred group of carriers includes lactose, trehalose, raffinose, maltodextrins, glycine, sodium citrate, tromethamine hydrochloride, human serum albumin, and mannitol.

Such carrier materials may be combined with the therapeutic polypeptide such as insulin peptide prior to dehydration such as spray drying, i.e., by adding the carrier material to the polypeptidesolution which is prepared for spray drying. In that way, the carrier material will be formed simultaneously with and as part of the protein particles.

Typically, when the carrier is formed by spray drying together with the polypeptide, the polypeptide will be present in each individual particle at a weight percent in the range from 5% to 95%, preferably from 20% to 80%. The remainder of the particle will primarily be carrier material (typically being from 5% to 95%, usually being from 20% to 80% by weight), but will also include buffer (s) may include other components as described above.

Non-aqueous insulin formulationsmay also be combined with other active components. For example, it may be desirable to combine small amounts of amylin or active amylin analogues in the insulin formulations to improve the treatment of diabetes. Amylin is a hormone which is secreted with insulin from the pancreatic 0-cells in normal (non-diabetic) individuals. It is believed that amylin modulates insulin activity in vivo, and it has been proposed that simultaneous administration of amylin with insulin could improve blood glucose control. Combining amylin with insulin in the compositions of the present invention will provide a particularly convenient product for achieving such simultaneous administration. Amylin may be combined with insulin at from 0.1% by weight to 10% by weight (based on the total weight of insulin in a dose), preferably from 0.5% by weight to 2.5% by weight. Amylin is available from commercial suppliers, such as Amylin Corporation, San Diego, California, and can be readily formulated in the compositions of the present invention. For example, amylin may be dissolved in aqueous or other suitable solutions together with the insulin, and optionally carriers, and the solution spray dried to produce the powder product.

A spray including the spray-dried polypeptide can be produced by forcing a suspension or solution of polypeptide through a nozzle under pressure. The nozzle size and configuration, the applied pressure, and the liquid feed rate can be chosen to achieve the desired output and particle size. An electrospray can be produced, for example, by an electric field in connection with a capillary or nozzle feed.

Advantageously, particles of e.g. insulin delivered by a sprayer have a particle size less than about 10 μm, for example in the range of about 1 μm to about 5 μm. Pharmaceutical compositions according to the present invention containing a dehydrated such as spray-dried polypeptidedissolved in a semi-polar protic organic solvent such as in a polyolmay also be administered parenterally to patients in need of such a treatment.

Parenteral administration may be performed by subcutaneous, intramuscular or intravenous injection by means of a syringe, optionally a pen-like syringe. Alternatively, parenteral administration can be performed by means of an infusion pump.

The preparations of the invention may be used in connection with pumps such as insulin pumps. The insulin pumpsmay be prefixed and disposable, or the insulin preparations may be supplied froma reservoir which is removable. Insulin pumps may be skinmounted, implanted or carried, and the path of the insulin preparation from the storage compartment of the pump to the patient may be more or less tortuous. Non-limiting examples of insulin pumps are disclosed in US 5,957,895, US 5, 858, 001, US4., 468, 221, US 4,468, 221, US 5, 957, 895, US 5,858, 001, US6,074,US US5, 858, 001, US 5,527,288, and US 6, 074, 369.

Injectable compositionsaccording to the present invention of the dehydrated such as spray-dried polypeptide can be prepared using the conventional techniques of the pharmaceutical industry which involve dissolving and mixing the ingredients in a semi-polar protic organic solventsuch as a polyolas appropriate to give the desired end product.

Currently, injection is the typical mode of administering a biologically active protein to the systemic circulation. The recipient may experience discomfort or pain by injection. For this reason and others there may be problems with patient compliance using injection as a mode of administration. One alternative to injection is the peroral administration of biologically active polypeptides. In the case of insulin, oral delivery may have advantages beyond convenience and compliance issues. Insulin absorbed in the gastrointestinal tract mimics the physiology of insulin secreted by the pancreas because both are released into the portal vein and carried directly to the liver. Absorption into the portal circulation maintains a peripheral-portal insulin gradient that regulates insulin secretion. In its first passage through the liver, roughly 60% of the insulin is retained and metabolized, thereby reducing the incidence of peripheral hyperinsulinemia, a factor in diabetes related systemic complications. A feared and not uncommon complication of insulin treatment and other oral antidiabetic agents is hypoglycemia.

However, peroral bioavailability of polypeptides is extremely low due to extensive proteolytic degradation in the gastrointestinal tract and low epithelial permeability. For example, enzymatic degradation studies have shown that native insulin is degraded extensively in the presence of trypsin and $\alpha$-chymotrypsin. Hence, stabilization of polypeptides against proteolytic degradation by protein engineering, such as pegylation and mutation of hot spots, is a commonly used strategy to improve oral bioavailability. Another strategy to reduce proteolytic degradation is to incorporate the polypeptide into nanoparticles, microparticles, microemulsions, microemulsion pre-concentrates, liposomes and the like. The drug is not only protected from the hostile environment in the stomach and gastrointestinal tract but also these particles might be taken up from the enteral route into the systemic circulation via the Peyers patches.

In one embodiment, the pharmaceutical non-aqueous compositionis a microemulsion pre-concentrate. This is a composition which spontaneously self emulsifies upon dilution with an aqueous medium, e.g. water or gastrointestinal juice into for example an oil-in-water (o/w) microemulsion. Microemulsions are being understood as being thermodynamically stable systems with a mean domain size of the emulsified phase in the lower nm range, for example between 20-400nm.

In general, the microemulsion pre-concentrate comprises one or more hydrophilic components, one or more lipophilic components, one or more non-ionic surfactants and/or co-surfactants and an active ingredient e.g. a therapeutic peptide or protein completely dissolved in the pre-concentrate mixture. In addition, the microemulsion pre-concentrate may, if appropriate, comprise conventional adjuvants and additives. These self (micro)-emulsifying pre-concentrates can be ingested with the expectation that a microemulsion forms in the gastrointestinal tract. The microemulsion pre-concentrate may be administered encapsulated in an enteric coated soft gelatine capsule. Many microemulsions have been reported to enhance the bioavailability of several therapeutic compounds after oral administration.

An example of a composition of a microemulsion pre-concentrate is:

a) at least one semi-polar protic organic solvent such as a polyol(e.g. propylene glycol and/or glycerol)
b) one or more hydrophilic components such as glycerol and/or propylene glycol
b) one or more lipophilic components such as oils (e.g. mono-diglyceridesand/or triglycerides)
c) one or more non-ionic surfactants such as poloxamers and/or polysorbates
d) one or more co-surfactants such as ethanol
e) a dehydrated therapeutically active polypeptide(e.g. insulin)
f) one or more additives such as antioxidants and/or stabilizers.

Pharmaceutical compositions according to the present invention containing a dehydrated such as a spray-dried therapeutically active polypeptidedissolved in a semi-polar protic organic solvent such as in a polyol may also be administered bucally or sublingually in the form of aerosolised spray to patients in need of such a treatment. The Oral-lyn™ formulation developed by Generex Biotechnology Corporation is an example of a buccal aerosol formulation administered via a pressurized metered dose inhaler (pMDI). Hydrofluoroalkanes (HFA), such as HFA 134a and 227, are typically used as propellants. In one embodiment the therapeutically active polypeptides according to the invention are typically highly soluble in water at pH values more than 1 unit below or above pI. Since water is extremely poorly miscible with HFA propellants it is advantageous to use a solvent such as propylene glycol and ethanol for the protein. Said solvent is miscible with HFA propellants. Solution-type metered dose inhalers are generally preferred relative to suspension-type pMDI, which tend to have a higher variation in dose uniformity due to creaming or sedimentation of particles.
The solution type pMDI may be formulated with stabilisers and other additives, such as permeation enhancers in addition to the therapeutically active polypeptide.

Instead of a pressurized system, the therapeutically active polypeptidemay alternatively be administered bucally or sublingually as a pumpable, such as the Coro Nitro Pump Spray® for sublingual administration of nitroglycerin.

In order to avoid lung deposition the oral spray aerosol should preferably have droplet diameters larger than 10 μm.

Mucoadhesive drug delivery systems have gained large interest in non-invasive protein/peptide delivery. They may increase the residence time of the delivery system on mucosal tissues. Several bioadhesive delivery systems have been reported to enhance the bioavailability of proteins and peptides for example after oral administration.

In a preferred embodiment, a non-aqueous freebioadhesive compositioncomprises aactive protein or peptide, at least one bioadhesive polymer, and one or more semi-polar protic organic solventsuch as a polyol(s). In addition, the composition may, if appropriate, comprise conventional adjuvants and additives. The bioadhesive polymer may for example be poly(ethylene oxide)-poly(propylene oxide)-poly(ethylene oxide) block copolymers (such as Poloxamers), polyvinylpyrrolidone (such as Povidone or PVP), polyacrylic acid, polycarbophil (such as Carbopol or Carbomer), chitosan, all kinds of thiolated polymers (such as Thiomers), cellulose, alginates, hydroxyethylcellulose or other bioadhesive polymers.

The composition can be a liquid, semi-solid or a solid dosage form.

These novel compositions would have the advantage compared to for example conventional bioadhesive hydrogels, that the required aqueous medium in these formulations can be avoided and thereof increased storage stability as well as increased bioavailabilty (triggered by the polyol) will be achieved.

An example of a bioadhesive composition:

a) a dehydrated therapeutically active polypeptide(e.g. insulin)
b) at least one semi-polar protic organic solvent such a polyol (e.g. propylene glycol and/or glycerol)
c) bioadhesive polymer (e.g. Povidone and/orPoloxamer)
d) Additives

In one embodiment, a non-aqueous composition contains a combination of a dehydrated therapeutically active polypeptide, at least one semi-polar protic organic solventsuch as polyol and one or more conventional permeation enhancers. Examples of permeation enhancers are but not limited to fatty acids, palmitoylcarnitine chloride, ethylene glycol-bis(beta-aminoethyl ether)-N,N,N',N'-tetraacetic acid (EGTA), EDTA, bile salts, ionic- as well as non-ionic surfactants such as phospholipids and alkylglycosides, polymers, chitosan, citric acid and sodium salicylate. In addition, the composition may, if appropriate, comprise conventional adjuvants and additives.

The compositions of the present invention can be administered in a format selected from e.g. a tablet, a capsule, a suppository, a liquid, a spray or an aerosol for buccal and peroral administration.

In a preferred embodiment, solid dosage forms based on semi-polar protic organic solvent(s) such aspolyols comprises at least one polyol and one or more at room temperature solid excipients for example a poloxamer, PEG or PEG stearate.

In another embodiment, the composition contains a dehydrated therapeutically active polypeptide, at least one semi-polar protic organic solventsuch as at least one polyol, and at least one at room temperature solid non ionic excipient for example poloxamers or polyoxyethylene glycols or a mixture of solid excipients. Examples of solid poloxamers are Pluronic F-127, Pluronic F-68. Examples of solid PEGs are PEG 3350, PEG 4000, PEG 8000. In addition, the solid composition may, if appropriate, comprise conventional adjuvants and additives such as binders, glidantsand lubricants to ensure efficient tabletting; and disintegrants to ensure that the tablet breaks up in the digestive tract.

Tablets and capsules may be coated with a entero coating, which ruptures or becomes permeable upon contacting an aqueous environment of a defined pH, make it possible for contents of the dosage format to be selectively released at a desired site in the gastro-intestinal tract (e.g. small and large intestine) by selecting the a suitable pH-soluble polymer for a specific region. Examples of suitable enteric polymers include but are not limited to cellulose acetate phthalate, hydroxypropylmethylcellulose phthalate, polyvinylacetate phthalate, hydroxypropylmethylcellulose phthalate, methacrylic acid copolymer, shellac, methylcellulose phthalate, cellulose acetate trimellitate, hydroxypropylmethylcellulose acetate succinate, cellulose acetate phthalate, cellulose acetate succinate, cellulose acetate malate, cellulose benzoate phthalate, cellulose propionate phthalate, carboxymethylethylcellulose, ethylhydroxyethylcellulose phthalate, shellac, styrene- acrylic acid copolymer, methyl acrylate-acrylic acid copolymer, methyl acrylate- methacrylic acid copolymer, butyl acrylate-styrene-acrylic acid copolymer, methacrylic acid-methyl methacrylate copolymer, methacrylic acid-ethyl acrylate copolymer, methyl acrylate-methacrylic acid-octyl acrylate copolymer, vinyl acetate-maleic acid anhydride copolymer, styrene-maleic acid anhydride copolymer, styrene-maleic acid monoester copolymer, vinyl methyl ether-maleic acid anhydride copolymer, ethylene-maleic acid anhydride copolymer, vinyl butyl ether-maleic acid anhydride copolymer, acrylonitrile- methyl acrylate-maleic acid anhydride copolymer, butyl acrylate-styrene-maleic acid anhydride copolymer, polyvinyl alcohol phthalate, polyvinyl acetal phthalate, polyvinyl butylate phthalate and polyvinyl acetoacetal phthalate, or combinations thereof.

In a preferred embodiment the dosage form is soft gelatine capsule containing a liquid.

The invention also relates to the following embodiments:

1. Pharmaceutical non-aqueous composition comprising a mixture of

    a) a dehydrated therapeutically active polypeptide preferably comprising 10-100 amino acids, and
    b) at least onesemi-polar protic organic solvent

whichpolypeptide has been dehydratedat a target pH which is at least 1 pH unit from the pIof the polypeptide in aqueous solution, and where said target pH preferably is in the range from about 6.0 to about 9.0.

2. Pharmaceutical non-aqueous composition comprising a mixture of

    a) a dehydrated therapeutically active polypeptide preferably comprising 10-100 amino acids, and
    b) at least onesemi-polar protic organic solvent

which polypeptide has been dehydratedat a target pH which is at least 1 pH unit from the pIof the polypeptide in aqueous solution, and where said target pH preferably is in the range from about 6.0 to about 9.0, with the proviso that the polypeptide is not insulinotropic peptide, GLP-1(7-37) or an analog or derivative thereof, or exendin or an analog or derivative thereof.

3. The pharmaceutical composition according to embodiment 1 or 2, wherein the organic solvent is selected from the group consisting of polyols.

4. The pharmaceutical composition according to embodiment 3, wherein the organic solvent is selected from the group consisting of diols and triols.

5. The pharmaceutical composition according to embodiment 4, wherein the organic solvent is selected from the group consisting of propylene glycol and glycerol.

6. The pharmaceutical composition according to embodiment 5, wherein the organic solvent is propylene glycol.

7. The pharmaceutical composition according to embodiment 5, wherein the organic solvent is glycerol.

8. The pharmaceutical composition according to any one of embodiments 1-7, wherein the polypeptide is spray dried.

9. The pharmaceutical composition according to any one of embodiments 1-7, wherein the polypeptide is freeze-dried.

10. The pharmaceutical composition according to any one of embodiments 1-9, wherein the polypeptide has been dehydrated at a pH which is at least 1.5 pH unit from the pIof the polypeptide.

11. The pharmaceutical composition according to any one of embodiments 1-10, wherein the polypeptide has been dehydrated at a pH which is at least 2 pH unit from the pIof the polypeptide.

12. The pharmaceutical composition according to any one of embodiments 1-11, wherein the polypeptide has been dehydrated at a pH which is at least 2.5 pH unit above the pIof the polypeptide.

13. The pharmaceutical composition according to any one of embodiments 1-12, wherein the solubility of dehydrated polypeptide in the organic solvent is at least 20 mg/ml.

14. The pharmaceutical composition according to any one of embodiments 1-12, wherein the solubility of dehydrated polypeptide in the organic solvent is at least 30 mg/ml.

15. The pharmaceutical composition according to any one of embodiments 1-12, wherein the solubility of dehydrated polypeptide in the organic solvent is at least 40 mg/ml.

16. The pharmaceutical composition according to any one of embodiments 1-12, wherein the solubility of dehydrated polypeptide in the organic solvent is at least 50 mg/ml.

17. The pharmaceutical composition according to any one of embodiments 1-12, wherein the solubility of dehydrated polypeptide in the organic solvent is at least 60 mg/ml.

18. The pharmaceutical composition according to any one of embodiments 1-17, wherein thetarget pH is in the range from about 6.5 to about 8.5.

19. The pharmaceutical composition according to any one of embodiments 1-17, wherein the target pH is in the range from about 7.2 to about 8.3.

20. The pharmaceutical composition according to any one of embodiments 1-17, wherein the target pH is in the range from about 7.0 to about 8.5.

21. The pharmaceutical composition according to any one of embodiments 1-20, wherein the organic solvent is present in an amount of at least 20% w/w.

22. The pharmaceutical composition according to any one of embodiments 1-20, wherein the organic solvent is present in an amount of at least 30% w/w.

23. The pharmaceutical composition according to any one of embodiments 1-20, wherein the organic solvent is present in an amount of at least 40% w/w.

24. The pharmaceutical composition according to any one of embodiments 1-20, wherein the organic solvent is present in an amount of at least 50% w/w.

25. The pharmaceutical composition according to any one of embodiments 1-20, wherein the organic solvent is present in an amount of at least 80% w/w.

26. The pharmaceutical composition according to any one of embodiments 1-25, which comprises less than 10% w/w water.

27. The pharmaceutical composition according to any one of embodiments 1-25, which comprises less than 5% w/w water.

28. The pharmaceutical composition according to any one of embodiments 1-25, which comprises less than 2% w/w water.

29. The pharmaceutical composition according to any one of embodiments 1-28, wherein the composition is for pulmonary, parenteral, nasal or oral treatment of diabetes or hyperglycaemia.

30. The pharmaceutical composition according to any one of embodiments 1-28, wherein the composition is for pulmonary treatment of diabetes or hyperglycaemia.

31. The pharmaceutical composition according to any one of embodiments 1-28, wherein the composition is for oral treatment of diabetes or hyperglycaemia.

32. The pharmaceutical composition according to any one of embodiments 1-31, wherein the polypeptide is water soluble.

33. The pharmaceutical composition according to any one of embodiments 1-32, wherein the polypeptide is selected from the group consisting of insulin peptides, amylin, amylin analogues, amylin derivatives, $\alpha$-MSH , $\alpha$-MSH analogues, $\alpha$-MSH derivatives and/or any combination thereof.

34. The pharmaceutical composition according to any one of claims 1-33, wherein the insulin peptide is an insulin analogue.

35. The pharmaceutical composition according to any one of embodiments 1-34, wherein the insulin peptide is an insulin analogue selected from the group consisting of AspB28 human insulin; LysB28ProB29 human insulin; LysB3GluB29 human insulin and A14GluB25HisdesB30 human insulin.

36. The pharmaceutical composition according to any one of embodiments 1-35, wherein the composition is adapted for pulmonary treatment, oral treatment, nasal treatment or buccal treatment.

37. The pharmaceutical composition according to any one of embodiments 1-35, wherein the composition is adapted for pulmonary use.

38. The pharmaceutical composition according to any one of embodiments 1-35, wherein the composition is adapted for nasal treatment.

39. The pharmaceutical composition according to any one of embodiments 1-35, wherein the composition is adapted for oral use.

40. The pharmaceutical composition according to any one of embodiments 1-35, wherein the composition is adapted for buccal use.

41. A method of treating diabetes in a patient in need of such a treatment, comprising administering to the patient a therapeutically effective amount of a pharmaceutical composition according to any one of the embodiments 1-40.

42. A method of treatment or prevention of hyperglycemia, type 2 diabetes, impaired glucose tolerance andtype 1 diabetesin a patient in need of such a treatment, comprising administering to the patient a therapeutically effective amount of a pharmaceutical composition according to any one of the embodiments 1-40.

43. A method of delaying or preventing disease progression in type 2 diabetesin a patient in need of such a treatment, comprising administering to the patient a therapeutically effective amount of a pharmaceutical composition according to any one of the embodiments 1-40.

44. Pharmaceutical composition according to any one of embodiments 1-40for use as a medicament for treatment or prevention of hyperglycemia, type 2 diabetes, impaired glucose tolerance andtype 1 diabetes.

45. Pharmaceutical composition according to any one of embodiments 1-40for use as a medicament for delaying or preventing disease progression in type 2 diabetes.

The following is a list of aspects further describing the invention:

1a. Pharmaceutical non-aqueous composition comprising a mixture of

    a) a dehydrated therapeutically active polypeptide comprising 10-100 amino acids, and

    b) at least onesemi-polar protic organic solvent

which polypeptide has been dehydratedat a target pH which is at least 1 pH unit from the pIof the polypeptide in aqueous solution.

2a. The pharmaceutical composition according to aspect 1a, wherein the organic solvent is selected from the group consisting of propylene glycol and glycerol.

3a. The pharmaceutical composition according to any one of aspects 1a-2a, wherein the solubility of dehydrated polypeptide in the organic solvent is at least 20 mg/ml.

4a. The pharmaceutical composition according to any one of aspects 1a-3a, wherein thetarget pH is in the range from about 6.0 to about 9.0.

5a. The pharmaceutical composition according to any one of aspects 1a-4a, wherein the organic solvent is present in an amount of at least 20% w/w.

6a. The pharmaceutical composition according to any one of aspects 1a-5a, which comprises less than 10% w/w water.

7a. The pharmaceutical composition according to any one of aspects 1a-6a, wherein the composition is adapted for pulmonary treatment, oral treatment, nasal treatment or buccal treatment.

8a. The pharmaceutical composition according to any one of aspects 1a-7a, wherein the polypeptide is selected from the group consisting of insulin peptides, amylin, amylin analogues, amylin derivatives, α-MSH , α-MSH analogues, α-MSH derivativesand/or any combination thereof.

9a. The pharmaceutical composition according to any one of aspects 1a-8a, wherein the insulin peptide is an insulin analogue.

10a. Pharmaceutical composition according to any one of aspects 1a-9afor use as a medicament for treatment or prevention of hyperglycemia, type 2 diabetes, impaired glucose tolerance and type 1 diabetes.

All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law).

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed.No language in the specification should be construed as indicating any non-claimed element as essential to the practice of the invention.

The citation and incorporation of patent documents herein is done for convenience only and does not reflect any view of the validity, patentability, and/or enforceability of such patent documents.

This invention includes all modifications and equivalents of the subject matter recited in the claims appended hereto as permitted by applicable law.

EXAMPLES

General procedure

Thioflavin T (ThT) fibrillation assay: Principle and examples

Low physical stability of a peptide may lead to amyloid fibril formation, which is observed as well-ordered, thread-like macromolecular structures in the sample eventually resulting in gel formation. This has traditionally been measured by visual inspection of the sample. However, that kind of measurement is very subjective and depending on the observer. Therefore, the application of a small molecule indicator probe is much more advantageous. Thioflavin T (ThT) is such a probe and has a distinct fluorescence signature when binding to fibrils [Naiki et al. (1989) Anal. Biochem. 177, 244-249; LeVine (1999) Methods.Enzymol. 309, 274-284].

Dynamic (accelerated) ThT assay

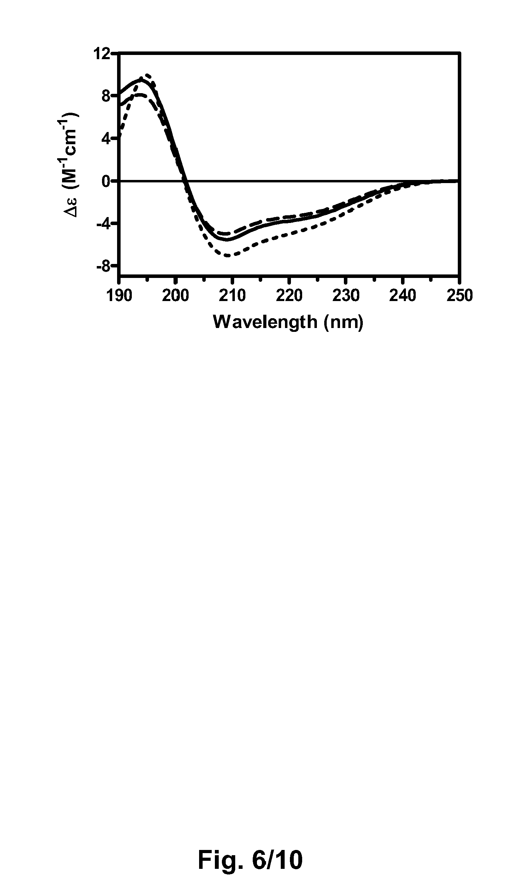

The time course for fibril formation can be described by a sigmoidal curve with the following expression [Nielsen et al. (2001) Biochemistry 40, 6036-6046], cn.f figure 6:

$$F = f_i + m_i t + \frac{f_f + m_f t}{1 + e^{-[(t-t_0)/\tau]}} \quad \text{Eq.(1)}$$

Here, F is the ThT fluorescence at the time t. The constant $t_0$ is the time needed to reach 50% of maximum fluorescence. The two important parameters describing fibril formation are the lag-time calculated by $t_0 - 2\tau$ and the apparent rate constant $k_{app} = 1/\tau$.

Formation of a partially folded intermediate of the peptide is suggested as a general initiating mechanism for fibrillation. Few of those intermediates nucleate to form a template onto which further intermediates may assembly and the fibrillation proceeds. The lag-time corresponds to the interval in which the critical mass of nucleus is built up and the apparent rate constant is the rate with which the fibril itself is formed.

Sample preparation

Samples were prepared freshly before each assay. Thioflavin T was added to the samples from a stock solution in $H_2O$ to a final concentration of 1 $\mu M$. Sample aliquots of 200 $\mu l$ were placed in a 96 well microtiter plate (Packard OptiPlate™-96, white polystyrene). Usually, eight replica of each sample (corresponding to one test condition) were placed in one column of wells. The plate was sealed with Scotch Pad (Qiagen).

Incubation and fluorescence measurement

Incubation at given temperature, shaking and measurement of the ThT fluorescence emission were done in a FluoroskanAscentFL fluorescence platereader (Thermo Labsystems). The temperature was adjusted to 37 °C. The orbital shaking was adjusted to 960 rpm with an amplitude of 1 mm in all the presented data. Fluorescence measurement was done using excitation through a 444 nm filter and measurement of emission through a 485 nm filter. Each run was initiated by incubating the plate at the assay temperature for 10 min. The plate was measured every 20 minutes for typically 45 hours. Between each measurement, the plate was shaken and heated as described.

Static ThT assay

Sample preparation and fluorescence measurement

Samples containing insulin aspart in non-aqueous propylene glycol was stored at 5 and 40°C for up to 1 month. The samples were prior to measurement diluted to an expected concentration (including fibrillated and native protein) of 1 mg/ml with demineralised water. Fluorescence measurements were performed in semimicro quartz cuvettes (Hellma, Germany) with a 0.3 cm excitation light path using a Perkin-Elmer model LS 50 B luminescence spectrometer. Emission spectra from 470 to 560 nm with excitation at 450 nm and with slit widths of 5 nm were recorded immediately after addition of 4 $\mu l$ ThT 1 mM stock solution to 196 $\mu l$ diluted sample. The concentration of ThT in reaction mixture was 20 $\mu Mol/l$. The results were reported as I 482 nm /[protein concentration (mg/ml) x cuvette path length (cm)], i.e. the results were normalized to a protein concentration of 1 mg/ml and a cuvette path length of 1 cm.

EXAMPLE 1

A) Dissolution of insulin human and adjustment of target pH to 7.0

9.5 g of insulin human was dispersed in 200 g ice cold water. The suspension was placed on ice bath and the initial pH was measured to pH 5.12. The pH was adjusted to 7.04 with 5.8 g of ice cold 0.2 N sodium hydroxide. The solution remained on ice bath for another 2 hour and then demineralised water was added to a total weight of 240 g. 50 g of the pH 7.04 solution was further processed. The pH was adjusted from 7.04 to 7.50 witch ice cold 1 N NH4OH and was then placed in a refrigerator overnight. The next day pH was measured to 7.32 and pH was adjusted with ice cold 1 N NH4OH to pH 8.06. Then water was added up to 60.0 g. The final concentration of insulin human was approximately 30 mg/ml.

B) Drying of aqueous insulin solution

Dry, solid microparticles were prepared on a Büchi B-290 mini spray dryer (Büchi, Labortechnik AG Flawil, Switzerland) equipped with a 0.7 mm co-current two-fluid nozzle. The insulin human solution was atomised into hot air stream in a drying chamber at a liquid feed rate of 2 ml/min and atomising air flow of 600-800 liter/hour. The drying air had an inlet temperature of 150°C and a drying air flow rate of 35 m3/hour. The outlet temperature was approximately 70°C. Solid microparticles were captured by a cyclone connected to the drying chamber and then gathered and stored at dry conditions.

C) Verification of target pH of solid insulin powder

Spray dried insulin human were re-dissolved in demineralised water at concentrations at 40 mg/mL, 80 mg/mL and 160 mg/mL to investigate if the different concentrations have influence of the measured pH value:

|  |  |
|---|---|
| 25.3 mg was added 633 µL water: | pH was measured to 6.95 |
| 43.5 mg was added 545 µL water: | pH was measured to 6.95 |
| 81.7 mg was added 510 µL water: | pH was measured to 7.01 |

EXAMPLE 2

Solubilization of insulin aspart with target pH ranging from pH 5.4 to pH 7.4 in 1,2propanediol (propylene glycol).
Various insulin aspart solutions with different target pH values were prepared prior to spray drying.
Solution A, Target pH 5.35:

16 g insulin aspart was suspended in 150 ml water on an ice bath. Next, 2.6 ml of ice cold concentrated aqueous ammonia (25 % w/w) was added stepwise until pH was 7.53 and a clear solution was obtained. Finally, water was added to a final concentration of insulin aspart of 40 mg/ml.

Solution B, Target pH 6.04:

16 g insulin aspart was suspended in 150 ml water on an ice bath. Initially, 2.2 ml of ice cold 1 N NaOH and then 750 µl ice cold concentrated aqueous ammonia (25 % w/w) was added stepwise until pH was 7.52 and a clear solution was obtained. Finally, water was added to a final concentration of insulin aspart of 40 mg/ml.

Solution C, Target pH 6.27:

16 g insulin aspart was suspended in 150 ml water on an ice bath. Initially, 4.4 ml of ice cold 1 N NaOH and then 750 µl ice cold concentrated aqueous ammonia (25 % w/w) was added stepwise until pH was 7.48 and a clear solution was obtained. Finally, water was added to a final concentration of insulin aspart of 40 mg/ml.

Solution D, Target pH 6.66:

16 g insulin aspart was suspended in 150 ml water on an ice bath. Initially, 6.6 ml of ice cold 1 N NaOH and then 370 µl ice cold concentrated aqueous ammonia (25 % w/w) was added stepwise until pH was 7.47 and a clear solution was obtained. Finally, water was added to a final concentration of insulin aspart of 40 mg/ml.

Solution E, Target pH 7.47:

16 g insulin aspart was suspended in 150 ml water on an ice bath. Initially, 9.6 ml of ice cold 1 N NaOH was added stepwise until pH was 7.49 and a clear solution was obtained.

Dry powders were prepared on a Büchi B-290 mini spray dryer (Büchi, Labortechnik AG Flawil, Switzerland) equipped with a 0.7 mm co-current two-fluid nozzle. The liquid feed (solution A, B, C, D and e) was atomised into hot air stream in a drying chamber at a liquid feed rate of 2 ml/min and at an atomising air flow of 600-800 liter/hour. The drying air had an inlet temperature of 150°C and a drying air flow rate of 35 m3/hour. The outlet temperature varied between 41 and 61°C.

Dry powders were captured by a cyclone connected to the drying chamber and then gathered and stored at dry conditions.

Moisture content of the dry microparticles was determined by loss on drying at 110°C for minimum 3 hours using a PerkinElmer Pyris TGA1 thermogravimetric analyzer. The weight change caused by moisture loss was registered and expressed in percent by weight

Target pH of the spray dried insulin was measured by dissolving the spray dried powders in demineralised water to a concentration of approximately 40 mg/ml and measuring pH by a potentiometer (Radiometer, Denmark).

Characterisation of spray dried powders:

| Solution | Moisture content (% w/w) | Target pH |
|---|---|---|
| A | 5.6 | 5.35 |
| B | 6.3 | 6.04 |
| C | 6.7 | 6.27 |
| D | 5.9 | 6.66 |
| E | 6.6 | 7.43 |

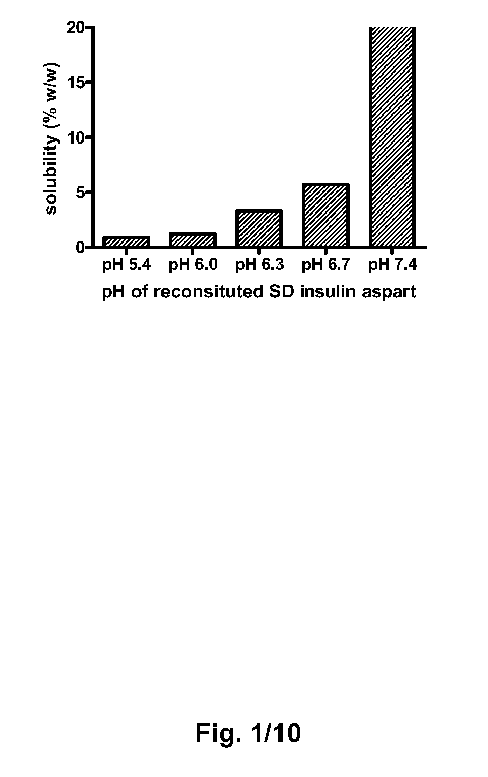

Solubility of the various insulin aspartpowders were measured by adding solid powder into a screw-cap vial (typical 200 mg of powder) followed by 2 g of 1,2 propanediol (propylene glycol). Powder and 1,2propanediol (propylene glycol)was shaken on a Swelab 820 mixer at 15 rpm (Boule Medical AB) at ambient temperature for at least 48 hours. More powder was added to the vial if the 1,2propanediol (propylene glycol)clarified. Then, non-dissolved insulin aspart was separated from solubilised insulin aspart by centrifugation at 4000 rpm for 0.5 hour. Insulin aspart concentration in the supernatant was measured by HPLC. Results from the HPLC analysis are shown in Figure 1.

EXAMPLE 3

Solubilization of insulin aspart with target pH of 7.5 in various organic semi-polar protic solvents.

Solubility of insulin Aspart powder with a target pH of 7.5in various organic semi-polar protic solvents (ethanediol (Ethylene Glycol]; 1,4butanediol; 1,3 butanediol; 1,3 propanediol, propanetriol (glycerol); 1,2 propanediol (propylene glycol)) was measured as described in Example 2.

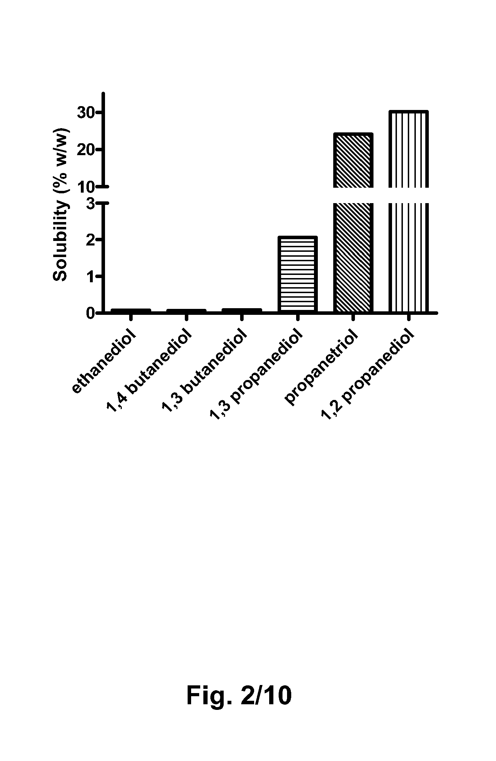

Results from the solubility study are shown in Figure 2.

EXAMPLE 4

In vitro evaluation of insulin aspart10 % propylene glycol formulation in rat gut sac model

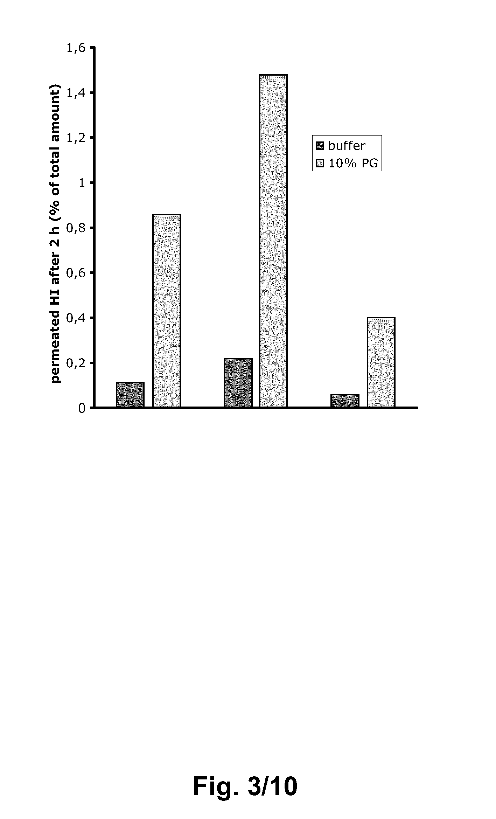

Freshly excised rat small intestine was rinsed in ice cold saline. Then gut sacs of 4 cm each in length were prepared. In brief, one end of the 4 cm gut segment was ligated with a string, then 0.25 ml of the insulin containing test solution (1 mM) was filled into the gut, and then the other end was ligated in the same way. Thereafter the filled gut sacs were incubated for 2 h in 0.5 ml culture medium pH 7.4 at 37°C. After 2h, the incubation medium was analysed by HPLC and MALDI for permeated insulin. Results from the HPLC analyses are shown in Figure 3. It can be observed that in presence of 10% PG, insulin permeates to a higher content across intestinal mucosa.

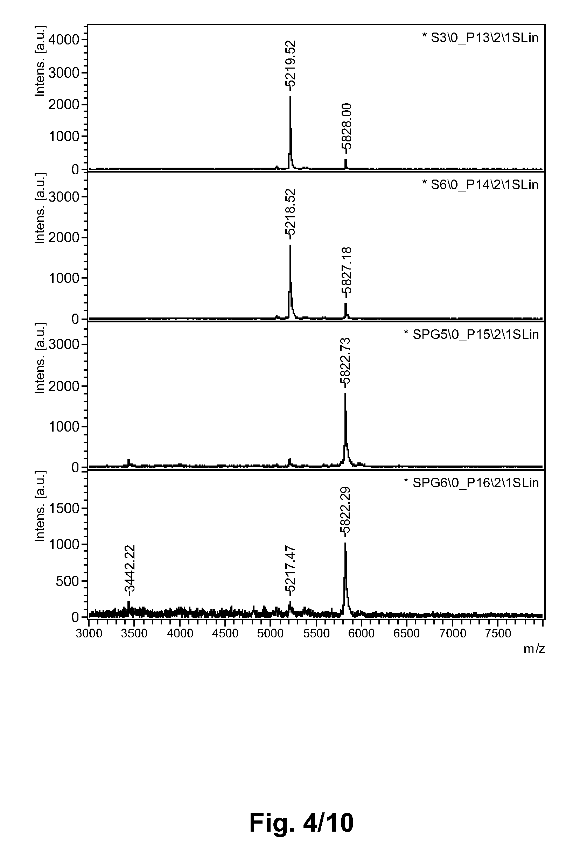

MALDI-MS (Matrix-assisted laser desorption/ionization-mass spectrometry) was used analyze degradation products. Aliquots (1 $\mu$l) of acidified sample solutions and reference samples were deposited on a PAC384 MALDI plate, blotted with filter paper after 30s and washed twice with 5 $\mu$l of 15 mM ammonium phosphate in 0.1% TFA.The samples were analyzed using AutoflexTofTof (BrukerDaltonics, USA). Results are shown in Figure 4. It seems that PG reduces the degradation of insulin in the gastrointestinal tract.

EXAMPLE 5

In vivo evaluation of insulin aspart propylene glycol formulation in rats following oral administration

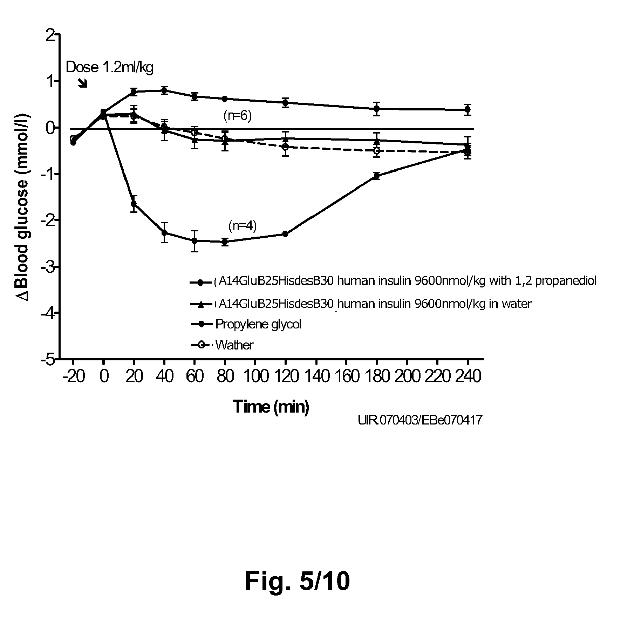

Sprague-Dawley rats were used. Rats were fasted before the experiment but had free access to drinking water. Rats were divided into 4 cohorts. Rats in 2 cohorts were gavaged with 1.2 ml/kg of A14GluB25HisdesB30 human insulin (8 mM) dissolved either in water or in 1,2 propanediol (propylene glycol). The 2 other cohorts received 1.2 ml/kg pure water or 1,2propanediol (propylene glycol) as control, respectively. In 20 minute intervals over a time period of 240 minutes, blood drops were collected from the tail vein and the glucose level was analysed. The results were expressed as Δ blood glucose (mmol/l). Results are shown in Figure 5.

EXAMPLE 6

Circular dichroism spectroscopy of Insulin Aspart in propylene glycol (PG)

Far-UV and near-UV circular dichroism measurements were used for monitoring for the conformational stability. Far-UV and near-UV circular dichroism spectra were obtained on a Jasco J-715 circular dichroism spectrophotometer (Jasco, Tokyo, Japan). The insulin samples were scanned in a 0.5 or a 0.02 cm cell using a bandwidth of 2.0 nm, a response time of 2 s, a data pitch of 0.5 nm, and a scanning speed of 20 nm/min. Spectra of the buffer were recorded and subtracted from each sample spectrum.

Far-UV spectra probes the peptide amide chromophore and can be used to estimates the protein secondary structure, hence the negative bands at 209 nm and 222 nm are indicative of α-helix structure. A slight change in intensity is observed in the spectrum of Aspart (Figure 6) in 100% PG as compared to that in 100% water, suggesting more α-helix structure in insulin in 100% PG. However, this difference did not seem significant when comparing different preparations of Aspart in PG. The spectra of Aspart in water and that of Aspart diluted to 2% PG from a sample of 100% PG are very similar showing that possible minor changes in α-helix content are reversible.

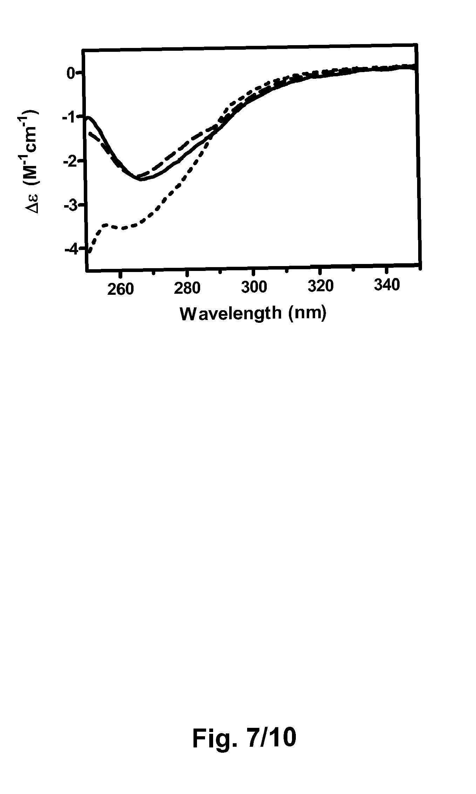

The CD spectrum in the 250-350 nm region reflects the local environment of tyrosine side chain residues in addition to disulphide bridge local environment. The far-UV spectrum (Figure 7) of insulin Aspart in 100% PG is very similar to that observed for insulins in denaturing solvents, e.gGuHCl, having increased amplitude at 250 nm and 270 nm. However when insulin in 100% PG is diluted in water to 2% PG the spectrum retains the shape of that observed for Insulin in pure water. This shows that despite effects of PG on side chain environment these structural effects are reversible.

Taking both the FUV and the NUC CD data into account, data show that secondary structure of the peptide backbone are more or less unchanged in 100% PG. Prominent changes in side chain environment of the same sample suggest the insulin taking up a so-called molten globule structure where back-bone folding is intact but side chain structure has collapsed. The structural changes are however reversible when Aspart in PG is diluted in water.

EXAMPLE 7

Storage stability of insulin aspartin propylene glycol solution vs. aqueous solution.

Dehydrated powder with a target pH of 7.5 of insulin aspart (IA) was dissolved in either neat propylene glycol or an aqueous 0.1 M TRIS buffer, pH 7.5. The concentration of insulin in the solutions was approximately 35 mg/ml. The solutions were incubated at ambient temperature and 40C for up to 40 weeks. The content of soluble covalent di-and polymer products was analyzed by size-exclusion chromatography (SEC). Samples were subjected to SEC at room temperature using a Waters insulin HMWP column 7.8×300 mm (Waters Corporation, Milford, MA, USA) at a flow rate of 1 ml/min with an eluent comprising 15 volumes of glacial acetic acid, 15 volumes of acetonitrile and 70 volumes of a 0.65 g/l solution of L-arginine. Detection was performed at 276 nm. The content of deamidation products and other insulin related substances was analyzed by reversed-phase high-performance liquid chromatography (RP-HPLC). The amount of fibrils in the samples was measured by the static ThT assay.

**Claims**

1.  Pharmaceutical non-aqueous composition comprising a mixture of

    a) a dehydrated therapeutically active polypeptide comprising 10-100 amino acids, and
    b) at least one semi-polar protic organic solvent
    which polypeptide has been dehydratedat a target pH which is at least 1 pH unit from the pI of the polypeptide in aqueous solution.

2.  The pharmaceutical composition according to claim 1, wherein the organic solvent is selected from the group consisting of propylene glycol and glycerol.

3.  The pharmaceutical composition according to any one of claims 1-2, wherein the solubility of dehydrated polypeptide in the organic solvent is at least 20 mg/ml.

4. The pharmaceutical composition according to any one of claims 1-3, wherein the target pH is in the range from about 6.0 to about 9.0.

5. The pharmaceutical composition according to any one of claims 1-4, wherein the organic solvent is present in an amount of at least 20% w/w.

6. The pharmaceutical composition according to any one of claims 1-5, which comprises less than 10% w/w water.

7. The pharmaceutical composition according to anyone of claims 1-30, wherein the composition is for pulmonary, parenteral, nasal or oral treatmentof diabetes or hyperglycaemia.

8. The pharmaceutical composition according to any one of claims 1-7, wherein the polypeptide is selected from the group consisting of insulin peptides, amylin, amylin analogues, amylin derivatives, $\alpha$-MSH , $\alpha$-MSH analogues, $\alpha$-MSH derivatives and/or any combination thereof.

9. The pharmaceutical composition according to any one of claims 1-8, wherein the insulin peptide is an insulin analogue.

10. Pharmaceutical composition according to any one of claims 1-9 for use as a medicament for treatment or prevention of hyperglycemia, type 2 diabetes, impaired glucose tolerance and type 1 diabetes.

11. A process for the preparation of a pharmaceutical solution by:

a) providing an aqueous solution of a therapeutically active polypeptide optionally comprising excipients,
b) adjusting the pH value to a target pH valuewhich is 1 unit, preferably 2 units and morepreferably 2.5 pH units above or below the pI of the polypeptide,
c) removingwater (dehydrating) the polypeptide by conventional drying technologies such as freeze- and spray drying, and
d) mixing and dissolution of the polypeptide in a semi-polar protic non-aqueous solvent e.g. by stirring, tumbling or othermixingmethods,
e) optionally filtration or centrifugation of the non-aqueouspolypeptide solution to remove non-dissolvedinorganic salts,
f) optionally removing residual amounts of waters by e.g. adding solid dessicants or vacuumdrying,
g) optionally adding further excipients such as a hydrofluoroalkanepropellants and cosolvents for solution pressurized metered dose inhalers or adding detergents, polymers, lipids and co-solvents for oral dosage forms.

12. A dehydrated pharmaceutically active polypeptide obtainable by the process of dehydrating the polypeptide at a target pH which is at least 1 pH unit from the pI of the polypeptide in aqueous solution.

13. The dehydrated pharmaceutically active polypeptide according to claim 12, wherein the solubility of said polypeptide is at least 20 mg/mL in organic solvent

14. The dehydrated pharmaceutically active polypeptide according to claim 13, whereinsaidorganic solvent is a semi-polar proticorganic solvent.

15. The dehydrated pharmaceutically active polypeptide according to anyone of the claims 12 - 14, wherein said organic solvent is a polyol.

Fig. 1/10

Fig. 2/10

Fig. 3/10

**Fig. 4/10**

Fig. 5/10

**Fig. 6/10**

**Fig. 7/10**

**Fig. 8/10**

Fig. 9/10

Fig. 10/10

Europäisches
Patentamt

European
Patent Office

Office européen
des brevets

**EUROPEAN SEARCH REPORT**

Application Number

EP 12 15 9419

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/047948 A (NASTECH PHARM CO [US]; QUAY STEVEN C [US]; COHEN ANNEMARIE STOUDT [US]) 26 April 2007 (2007-04-26)<br>* paragraphs [0041], [0053], [0055] - [0057], [0120], [0163] *<br>* page 7, line 11 - line 37 *<br>* page 14, line 3 - page 16, line 15 *<br>* page 53, line 14 - line 28 *<br>----- | 1-15 | INV.<br>A61K9/00<br>A61K9/08<br>A61K9/19<br>A61K38/04<br>A61K38/16<br>A61K47/10<br>A61K47/42<br>A61K9/12<br>A61K38/28 |
| X | US 5 981 489 A (STEVENSON CYNTHIA L [US] ET AL) 9 November 1999 (1999-11-09)<br>* column 3, line 22 - line 55; claim 1 *<br>----- | 1-15 | |
| X | WO 2005/115441 A2 (NASTECH PHARM CO [US]; QUAY STEVEN C [US]; COSTANTINO HENRY R [US]; KL) 8 December 2005 (2005-12-08)<br>* page 3, line 26 - page 5, line 19; claims 21,25,28, *<br>----- | 1-15 | |
| X | WO 94/19020 A1 (GENENTECH INC [US]; CLELAND JEFFREY L [US]; JONES ANDREW J S [US]) 1 September 1994 (1994-09-01)<br>* page 2, line 16 - page 3, line 20; claims 1,9,18 *<br>----- | 1-15 | TECHNICAL FIELDS SEARCHED (IPC)<br><br>A61K |

The present search report has been drawn up for all claims

| Place of search | Date of completion of the search | Examiner |
|---|---|---|
| Munich | 12 September 2012 | Giese, Hans-Hermann |

EPO FORM 1503 03.82 (P04C01)

# EP 2 514 407 A1

**ANNEX TO THE EUROPEAN SEARCH REPORT
ON EUROPEAN PATENT APPLICATION NO.**

EP 12 15 9419

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2012

| Patent document cited in search report | | Publication date | Patent family member(s) | | Publication date |
|---|---|---|---|---|---|
| WO 2007047948 | A | 26-04-2007 | AT | 471144 T | 15-07-2010 |
| | | | AU | 2006304757 A1 | 26-04-2007 |
| | | | CA | 2626357 A1 | 26-04-2007 |
| | | | EP | 1951198 A2 | 06-08-2008 |
| | | | JP | 2009512709 A | 26-03-2009 |
| | | | KR | 20080064171 A | 08-07-2008 |
| | | | US | 2010210506 A1 | 19-08-2010 |
| | | | WO | 2007047948 A2 | 26-04-2007 |
| US 5981489 | A | 09-11-1999 | US | 5981489 A | 09-11-1999 |
| | | | US | 6066619 A | 23-05-2000 |
| WO 2005115441 | A2 | 08-12-2005 | AU | 2005247369 A1 | 08-12-2005 |
| | | | CA | 2567056 A1 | 08-12-2005 |
| | | | CN | 101151048 A | 26-03-2008 |
| | | | EP | 1750756 A2 | 14-02-2007 |
| | | | JP | 2007537274 A | 20-12-2007 |
| | | | US | 2005276843 A1 | 15-12-2005 |
| | | | US | 2007154400 A1 | 05-07-2007 |
| | | | US | 2007154401 A1 | 05-07-2007 |
| | | | US | 2007167364 A1 | 19-07-2007 |
| | | | US | 2007244049 A1 | 18-10-2007 |
| | | | US | 2010184688 A1 | 22-07-2010 |
| | | | WO | 2005115441 A2 | 08-12-2005 |
| WO 9419020 | A1 | 01-09-1994 | AT | 197550 T | 15-12-2000 |
| | | | AU | 685784 B2 | 29-01-1998 |
| | | | AU | 6241294 A | 14-09-1994 |
| | | | CN | 1118143 A | 06-03-1996 |
| | | | CZ | 9502127 A3 | 14-02-1996 |
| | | | DE | 69426292 D1 | 21-12-2000 |
| | | | DE | 69426292 T2 | 17-05-2001 |
| | | | DK | 686045 T3 | 05-03-2001 |
| | | | EP | 0686045 A1 | 13-12-1995 |
| | | | ES | 2153418 T3 | 01-03-2001 |
| | | | GR | 3035383 T3 | 31-05-2001 |
| | | | IL | 108713 A | 22-12-1999 |
| | | | JP | 3698721 B2 | 21-09-2005 |
| | | | JP | H08507064 A | 30-07-1996 |
| | | | NZ | 262634 A | 24-02-1997 |
| | | | PT | 686045 E | 30-04-2001 |
| | | | RU | 2143889 C1 | 10-01-2000 |
| | | | US | 5589167 A | 31-12-1996 |
| | | | US | 5753219 A | 19-05-1998 |
| | | | US | 5804557 A | 08-09-1998 |
| | | | WO | 9419020 A1 | 01-09-1994 |

For more details about this annex : see Official Journal of the European Patent Office, No. 12/82

40

**ANNEX TO THE EUROPEAN SEARCH REPORT
ON EUROPEAN PATENT APPLICATION NO.** EP 12 15 9419

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2012

| Patent document cited in search report | Publication date | Patent family member(s) | | Publication date |
|---|---|---|---|---|
| | | ZA | 9401239 A | 23-08-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0042993 A
- WO 2008034881 A
- EP 0792290 A
- EP 0214826 A
- EP 0705275 A
- US 5504188 A
- EP 0368187 A
- US 5750497 A
- US 6011007 A
- EP 375437 A
- EP 383472 A
- WO 9507931 A
- WO 2005054291 A
- WO 2006018450 A
- WO 03078538 A
- US 5957895 A
- US 5858001 A
- US 4468221 A
- US 4468 A
- US 221 A
- US 5858 A
- US 001 A
- US 6074 A
- US 5527288 A
- US 6074369 A

### Non-patent literature cited in the description

- **J. T. CHIN ; S. L. WHEELER ; A. M. KLIBANOV.** Communication to the editor: On protein solubility in organic solvents. *BIOTECHNOL.BIOENG,* 1994, vol. 44 (1), 140-145
- **HUDSON ; ANDERSEN.** *Peptide Science,* 2004, vol. 76 (4), 298-308
- **GREENE ; WUTS.** Protective Groups in Organic Synthesis. John Wiley & Sons, 1999
- **NAIKI et al.** *Anal. Biochem.,* 1989, vol. 177, 244-249
- **LEVINE.** *Methods.Enzymol.,* 1999, vol. 309, 274-284
- **NIELSEN et al.** *Biochemistry,* 2001, vol. 40, 6036-6046

**SEQUENCE LISTING**

```
<110>  Novo Nordisk A/S

<120>  Stable non-aqueous pharmaceutical compositions

<130>  7634.000-EP

<160>  2

<170>  PatentIn version 3.4

<210>  1
<211>  31
<212>  PRT
<213>  Homo Sapiens

<400>  1

His Ala Glu His Thr Phe Thr Ser Asp Val Ser Ser Tyr Leu Glu Gly
1               5               10              15


Gln Ala Ala Lys Glu Phe Ile Ala Trp Leu Val Lys Gly Arg Gly
            20              25              30


<210>  2
<211>  39
<212>  PRT
<213>  Heloderma suspectum

<400>  2

His Gly Glu Gly Thr Phe Thr Ser Asp Leu Ser Lys Gln Met Glu Glu
1               5               10              15


Glu Ala Val Arg Leu Phe Ile Glu Trp Leu Lys Asn Gly Gly Pro Ser
            20              25              30


Ser Gly Ala Pro Pro Pro Ser
            35
```